# EUROPEAN PATENT APPLICATION

(11) **EP 4 431 050 A1**
(43) Date of publication of application: **18.09.2024**
(21) Application number: 21969910.5
(22) Date of filing: 27.12.2021
(51) Int. Cl.: A61B 34/37, B25J 3/00

(54) **SURGERY ASSISTING DEVICE**

(71) Applicant: RIVERFIELD Inc., Tokyo 107-0052 (JP)
(72) Inventor: KANAZAWA, Masao, Tokyo 160-0017 (JP); TADANO, Kotaro, Tokyo 160-0017 (JP)
(74) Representative: adares Patent- und Rechtsanwälte Reininger & Partner GmbB
(86) International application number: PCT/JP2021/048580
(87) International publication number: WO 2023/127026

(57) **Abstract**

A surgery assisting device according to the present invention is a master-side device for remotely manipulating a slave-side device, and includes: a first manipulator having a predetermined range of movement and used to manipulate a movable part of the slave-side device; and a control unit configured to control the movable part to turn in a roll direction in response to a specific manipulation on the first manipulator, wherein the control unit performs processing of determining where or not to turn the movable part in the roll direction in response to the specific manipulation.

## Description

### Technical Field

The present invention relates to a technique for a surgery assisting device, and particularly to a technique for a surgery assisting device in which a master-side device and a slave-side device are linked to perform surgery on a patient.

### Background Art

As a device used by a surgeon to perform surgery on a patient, a master-side device that is linked with a slave-side device is known (e.g., PTL 1).

### Citation List

### Patent Literature

[PTL 1] Japanese Unexamined Patent Application Publication No. H08-215211

### Summary of Invention

### Technical Problem

With this type of device, the surgery proceeds with linking the movements of the surgeon's hands to the movements of surgical instruments such as forceps placed on the patient's side to cause the surgical instruments to move as intended by the surgeon.

However, the range of movement of the surgeon's hand is limited, and in some cases, the surgical instrument may not be able to be moved as intended.

The present invention has been made in view of the above circumstances, and an object of the present invention is to provide a surgery assisting device that can perform surgery appropriately.

### Solution to Problem

A surgery assisting device according to the present invention is a master-side device for remotely manipulating a slave-side device, and includes: a first manipulator having a predetermined range of movement and used to manipulate a movable part of the slave-side device; and a control unit configured to control the movable part to turn in a roll direction in response to a specific manipulation on the first manipulator, wherein the control unit performs processing of determining where or not to turn the movable part in the roll direction in response to the specific manipulation.

This makes it possible to remove the linkage between the master-side device 1 and the slave-side device 2. In particular, a configuration is provided in which the linkage can be removed only for translational motion in a predetermined direction or turning motion in a direction around a predetermined axis, making it possible to provide manipulation modes that meet various needs of the surgeon.

### Advantageous Effects of Invention

According to the present invention, it is possible to provide a surgery assisting device that can perform surgery appropriately.

### Brief Description of Drawings

Fig. 1 is a perspective view of a master-side device according to an embodiment of the present invention.
Fig. 2 is a perspective view of a slave-side device.
Fig. 3 is a perspective view of a right manipulation unit.
Fig. 4 is a perspective view of a left manipulation unit.
Fig. 5 is a perspective view illustrating a state (reference position) in which each movable part of a delta mechanism is located approximately at the center in the corresponding range of movement.
Fig. 6 is a perspective view illustrating a state in which a support part of the delta mechanism is located forward (on the surgeon's side) of the reference position.
Fig. 7 is a perspective view illustrating a state in which the support part of the delta mechanism is located above the reference position.
Fig. 8 is a perspective view conceptually illustrating an attitude changing mechanism.
Fig. 9 is a perspective view schematically illustrating the movement of the attitude changing mechanism in a turning manipulation in the pan direction.
Fig. 10 is a perspective view schematically illustrating the movement of the attitude changing mechanism in a turning manipulation in the pitch direction.
Fig. 11 is a perspective view schematically illustrating the movement of the attitude changing mechanism in a turning manipulation in the roll direction.
Fig. 12 is a side view mainly illustrating a grasping mechanism of the attitude changing mechanism.
Fig. 13 is a cross-sectional view illustrating a trocar placed on an abdominal wall of a patient and a surgical instrument inserted into the trocar.
Fig. 14 is a functional block diagram of a surgery assisting system.
Fig. 15 illustrates a state in which a first setting tab is selected on a setting screen displayed on a second monitor.
Fig. 16 illustrates a state in which a second setting tab is selected on the setting screen displayed on the second monitor.
Fig. 17 is an example of a flowchart for implementing a neutral assistance function.
Fig. 18 is an example of a flowchart for implementing a wrist angle scaling function.
Fig. 19 is an example of a flowchart for implementing a roll clutch function.
Fig. 20 illustrates a modification example of a state in which the second setting tab is selected on the setting screen displayed on the second monitor.

### Description of Embodiments

### <1. Configuration of Surgery Assisting System>

A surgery assisting system S according to an embodiment will be described below with reference to the attached drawings.

Note that each configuration described in the drawings referred to is merely an example for realizing the present invention. Therefore, various changes can be made depending on, for example, the design without departing from the technical idea of the present invention. Further, in order to avoid duplicate explanation, configurations that have been described once may be given the same reference numerals and repeated explanations may be omitted.

The surgery assisting system S includes a master-side device 1 and a slave-side device 2.

The master-side device 1 is a device used by a surgeon (doctor) who performs surgery. Further, the slave-side device 2 is a device that is installed in an operating room where a patient to be operated on is lying down, and performs an actual surgery on the patient.

The master-side device 1 and the slave-side device 2 are installed apart from each other. The master-side device 1 and the slave-side device 2 may be installed in separate rooms, or may be installed in the same room.

The master-side device 1 and the slave-side device 2 are connected to each other to enable wired or wireless data communication, and each part of the remotely located slave-side device 2 is driven to perform surgery on the patient in accordance with a surgeon's manipulation on the master-side device 1.

In the embodiment described below, an example is described in which the master-side device 1 and the slave-side device 2 are installed and used on the floor of an operating room. However, the scope in which the present invention is applied is not limited to the type that is installed and used on the floor of an operating room, and the present invention can also be applied to the type that is attached to and used on the ceiling or wall of an operating room.

Note that the front and back, up and down, and left and right directions described below are for convenience of explanation, and the implementation of the present invention is not limited by these directions.

Further, the surgery assisting system S described below has a configuration in which a movable body turns, but this "turning" means a movement in the direction around a reference axis (central axis) that includes any one point, and refers to a motion that also includes "rotating". Note that although an example is described below in which a movable body turns, the movable body is not limited to performing a turning motion, and may also perform a straight motion, and a "motion" means both a turning motion and a straight motion.

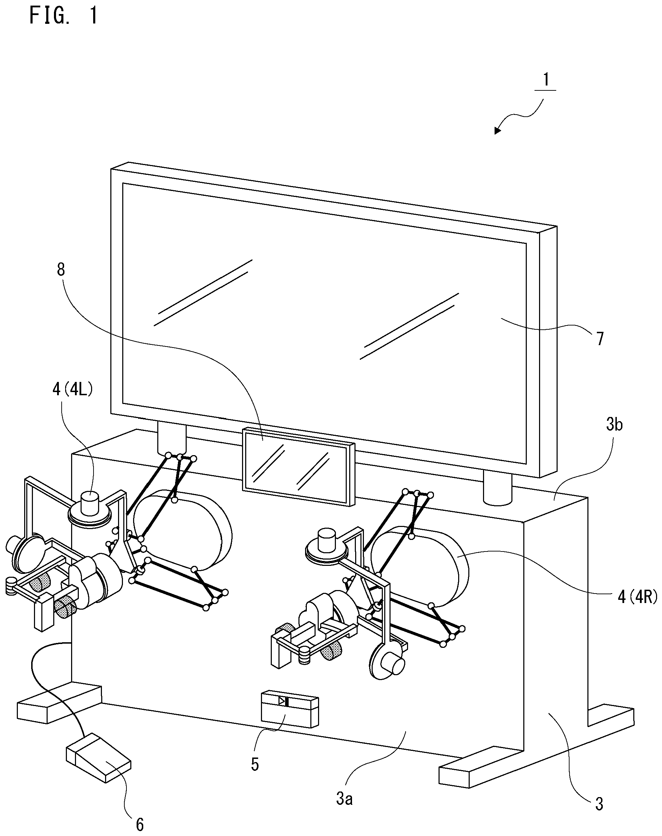

A specific configuration of the master-side device 1 is illustrated in Fig. 1.

Note that in the explanation of the master-side device 1, the front and back, and left and right directions are described supposing that the direction of the surgeon viewed from the master-side device 1 is the front. In other words, the surgeon is located in front of the master-side device 1.

The master-side device 1 includes a base part 3 that is installed on the floor, two manipulation units 4 that are attached apart from each other in the left and right above a front face part 3a of the base part 3, a first foot button 5 that is attached at the center below the front face part 3a, a second foot button 6 that is connected to the base part 3 by wire and can be placed on the floor, a first monitor 7 that is attached to a top face part 3b of the base part 3, and a second monitor 8 that is attached to the top face part 3b and is smaller than the first monitor 7.

One of the two manipulation units 4 is a right manipulation unit 4R that is handled by the surgeon's right hand, and the other is a left manipulation unit 4L that is handled by the surgeon's left hand.

The surgeon manipulates the right manipulation unit 4R with the right hand and the left manipulation unit 4L with the left hand while sitting on a chair (not illustrated) placed in front of the base part 3 (on the front face part 3a side).

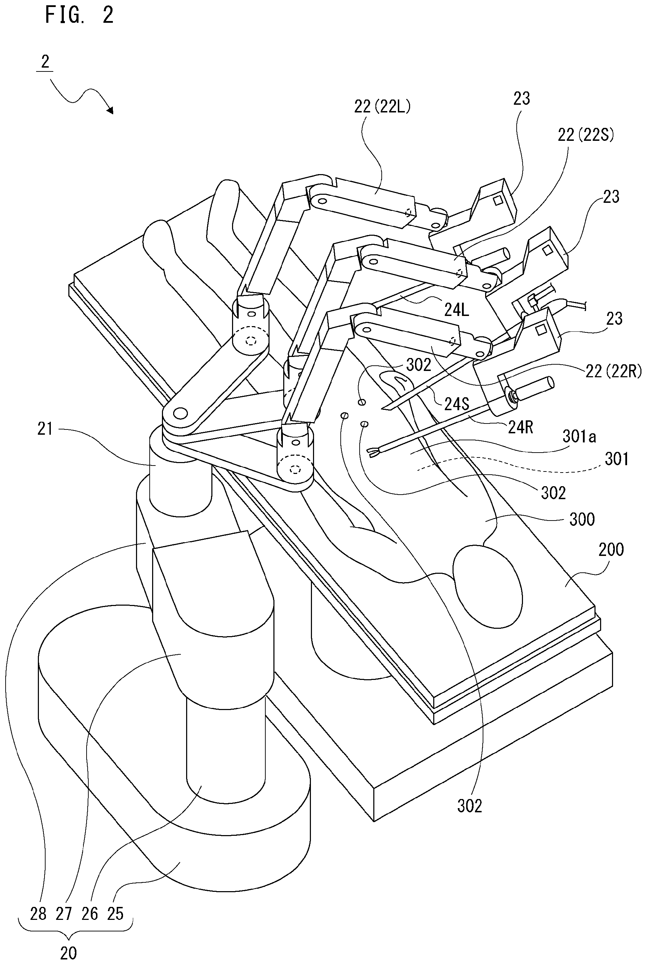

In response to manipulations using the right manipulation unit 4R and the left manipulation unit 4L, each part/unit of the slave-side device 2 illustrated in Fig. 2 is driven to perform surgery on the patient. The specific structures and manipulations of the right manipulation unit 4R and the left manipulation unit 4L, and the operation of the slave-side device 2 in response to the manipulations will be described later.

The first foot button 5 is a manipulator for switching whether or not to link a manipulation on the master-side device 1 with the operation of the slave-side device 2. Since the surgeon uses both hands to manipulate the manipulation units 4, it is desirable that the first foot button 5 be installed at a position where the surgeon can manipulate it with the foot.

Each time the first foot button 5 is manipulated, the master-side device 1 and the slave-side device 2 are switched between a state where they are linked and a state where they are not linked.

Note that the state where both devices are linked refers to a state where each part of the slave-side device 2, such as forceps, is driven in response to a manipulation on the manipulation units 4 of the master-side device 1. Further, the state where both devices are not linked refers to a state where each part of the slave-side device 2, such as forceps, is not driven and the position and attitude of each part are maintained even when the manipulation units 4 of the master-side device 1 are manipulated.

The second foot button 6 functions as an image capture button for capturing the image of a surgical field, such as the inside of the patient's body cavity. For example, the slave-side device 2 includes an endoscope in addition to the forceps manipulated by the right manipulation unit R and the forceps manipulated by the left manipulation unit 4L. The second foot button 6 is an image capture button for capturing and saving an image based on the viewing angle of the endoscope.

The first monitor 7 is a monitor device for displaying a live view image through the endoscope included in the slave-side device 2.

The second monitor 8 is a monitor device for displaying various types of information, and can display attitude information of the endoscope and various setting screens, for example. Screens to be displayed on the second monitor 8 will be described later.

The first monitor 7 and the second monitor 8 are each, for example, a display device such as a liquid crystal display (LCD) or an organic electro-luminescence (EL) display.

Next, the general configuration of the slave-side device 2 will be described with reference to Fig. 2. An operating table 200 is installed in the operating room, and a patient 300 is lying on the operating table 200, for example, in a supine state. One or more ports 302 are formed in a portion forming a body cavity 301 of the patient 300, for example, an abdominal wall 301a. A part (distal end part) of a surgical instrument, which will be described later, is inserted into each port 302 when a surgical operation is performed. The port 302 is a small hole into which a shaft-shaped surgical instrument is inserted.

The slave-side device 2 includes a base unit 20 that is placed on the floor or the like of the operating room, a stage 21 that is attached to the base unit 20, one or more arms 22 that are attached to the stage 21, surgical instrument holding devices 23 that are attached to the distal ends of the arms 22, respectively, and surgical instruments 24 that are detachably held by the surgical instrument holding devices 23, respectively.

The base unit 20 includes a base part 25 that is placed on the floor or the like of the operating room, a lifting mechanism 26 that is attached to the top of the base part 25, a base arm rear 27 that is attached to the top of the lifting mechanism 26, and a base arm front 28 that is attached to the base arm rear 27 so as to be extendable therefrom in the horizontal direction.

The lifting mechanism 26 extends and contracts in the vertical direction to elevate and lower the base arm rear 27 in the vertical direction so that the base arm rear 27 can be adjusted to an appropriate height position.

The base arm front 28 can extend from the base arm rear 27 in the horizontal direction so that the position of the stage 21 attached to the distal end of the base arm front 28 can be adjusted.

The stage 21 is formed on the base arm front 28 to pivotally support the arms 22.

In the present embodiment, three arms 22 are attached as the arms 22. One arm is a right arm 22R that holds a surgical instrument 24R that is driven in response to a manipulation on the right manipulation unit 4R of the master-side device 1, another arm is a left arm 22L that holds a surgical instrument 24L that is driven in response to a manipulation on the left manipulation unit 4L of the master-side device 1, and the remaining arm 22S is an endoscope arm 22S that holds an endoscope 24S as the surgical instrument 24. Note that although a configuration is illustrated by way example in which the endoscope 24S is provided as the surgical instrument 24, one of the surgical instruments 24 may be an imaging device other than an endoscope.

In the following description, when the term "surgical instrument 24" is used without specific explanation, it includes not only the surgical instruments 24R and 24L such as forceps but also the endoscope 24S.

Each arm 22 is turnable in a direction around an axis extending vertically on the stage 21.

Each arm 22 includes one or more joints and turning parts, and has a mechanism for easily moving its distal end to any position.

The surgical instrument holding device 23 is attached to the distal end part of the arm 22 through a gimbal mechanism or the like.

The endoscope 24S is, for example, provided as an endoscope unit having an endoscope, and includes a shaft extending back and forth, a camera head connected to the distal end part of the shaft, and a light guide connected to the middle part of the shaft.

The distal end part of the shaft of the surgical instrument 24 is inserted into the body cavity 301 through any one of the ports 302 formed in the patient 300.

In a state where the distal end part of the shaft is inserted into the body cavity 301, illumination light is emitted from the distal end part of the shaft of the endoscope unit, and the state inside the body cavity 301 is captured by an imaging element. Acquisition of an image based on that capture is executed, for example, by the second foot button 6 illustrated in Fig. 1.

The internal state of the body cavity 301 captured by the image sensor is transmitted as a captured image signal to the first monitor 7 illustrated in Fig. 1, making it possible for the surgeon to remotely observe the internal state of the body cavity 301.

Further, the surgeon can remotely manipulate the position and attitude of the surgical instrument 24R attached to the right arm 22R by manipulating the right manipulation unit 4R. Further, the surgeon can remotely manipulate the position and attitude of the surgical instrument 24L attached to the left arm 22L by manipulating the left manipulation unit 4L.

All or some of movable parts such as joints and turning parts of each arm 22 are driven by actuators such as built-in motors in response to a remote manipulation using the master-side device 1.

Note that although the slave-side device 2 is illustrated by way of example in which three surgical instruments 24 can be manipulated by the three arms 22, considering that the slave-side device 2 is used for surgery by a remote manipulation using the master-side device 1, any device may be used as long as it can handle the endoscope 24S and other surgical instruments 24 with two arms.

Further, in that case, the surgical instruments 24 other than the endoscope 24S may be remotely manipulated by the right manipulation unit 4R, remotely manipulated by the left manipulation unit 4L, or remotely manipulated by both the right manipulation unit 4R and the left manipulation unit 4L.

By using such a surgery assisting system S, it is possible to perform a surgical operation by remotely manipulating one or more surgical instruments, thereby making it possible to shorten the surgical time and also making it possible to perform advanced surgical operations using different types of surgical instruments.

### <2. Linkage between Manipulation Unit and Surgical Instruments>

The linkage between the manipulation unit 4 of the master-side device 1 and the surgical instrument 24 of the slave-side device 2 will be described with reference to the attached drawings.

The configuration of the manipulation unit 4 of the master-side device 1 will be described first.

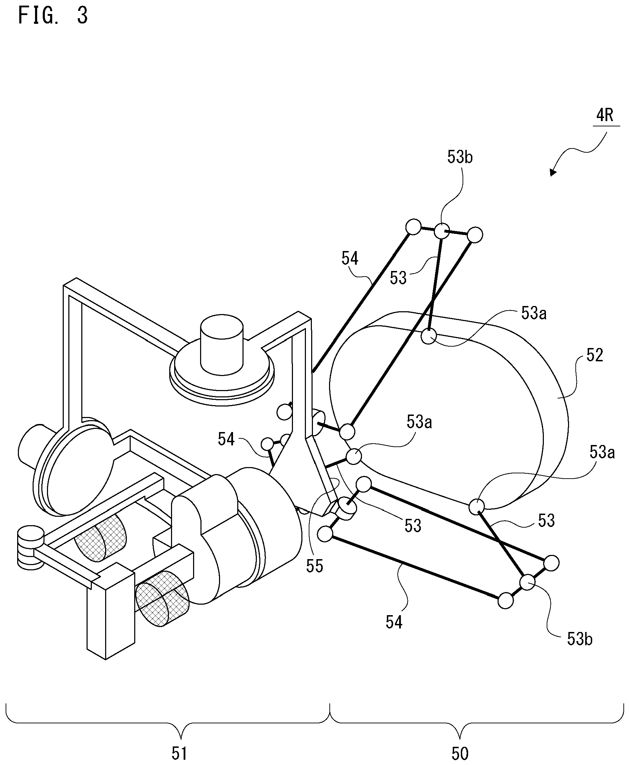

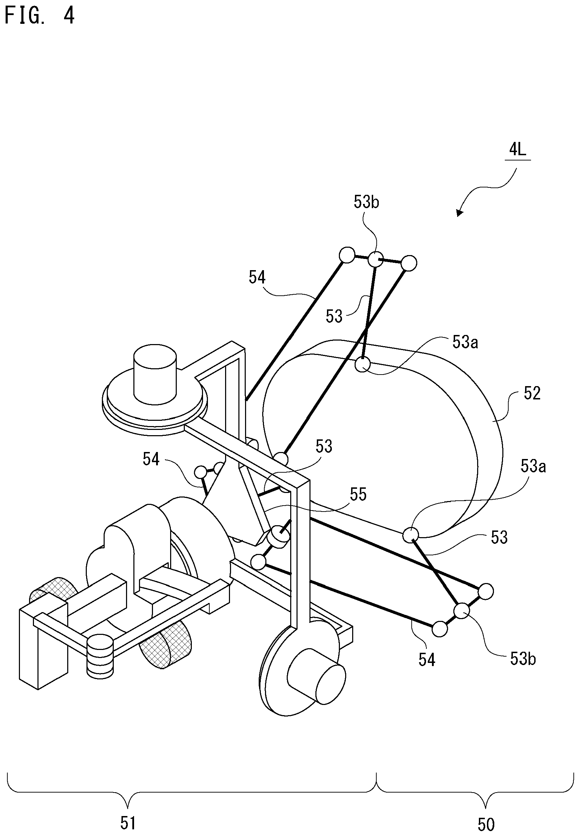

Fig. 3 is a perspective view of the right manipulation unit 4R, and Fig. 4 is a perspective view of the left manipulation unit 4L.

The manipulation unit 4 includes a delta mechanism 50 for moving the position of the surgical instrument 24 and an attitude changing mechanism 51 for changing the attitude of the surgical instrument 24.

The right manipulation unit 4R has a structure in which the left manipulation unit 4L is flipped horizontally. Therefore, in the following description, the left manipulation unit 4L will be mainly described.

The delta mechanism 50 has one end attached to the front face part 3a of the base part 3, and the other end serving as a support part that supports the attitude changing mechanism 51.

The delta mechanism 50 is a mechanism for positioning the corresponding surgical instrument 24 in the up and down, left and right, and front and back directions.

Note that in the following description, when describing the direction of the surgical instrument 24, the direction when viewed in the longitudinal direction (direction in which the axis extends) of the surgical instrument 24 will be described. Specifically, the front and back direction of the surgical instrument 24 refers to the longitudinal direction of the surgical instrument 24, the up and down direction of the surgical instrument 24 refer to the vertical direction in the reference attitude of the surgical instrument 24, and the left and right direction of the surgical instrument 24 refers to a direction perpendicular to the front and back direction and the vertical direction in the reference attitude of the surgical instrument 24.

Therefore, when the attitude of the surgical instrument 24 is rotated by 180 degrees around the axis extending in the longitudinal direction with respect to the reference attitude, that is, when the attitude of the surgical instrument 24 is upside down, the up and down directions of the surgical instrument 24 are reversed.

The surgeon grasps a predetermined part of the attitude changing mechanism 51 supported by the support part of the delta mechanism 50 and moves the entire attitude changing mechanism 51 in the up and down, left and right, and front and back directions, thereby turning each part of the delta mechanism 50 to position the surgical instrument 24.

Specifically, as illustrated in Figs. 3, 4, 5, and others, the delta mechanism 50 includes an attaching base part 52 serving as a base attached to the front face part 3a of the base part 3, three joint parts 53 having one ends attached to the attaching base part 52 at slightly above the center, slightly lower right of the center, and slightly lower left of the center, respectively, link mechanisms 54 attached to the other ends of the joint parts 53, and a support part 55 supported by the three link mechanisms 54 by being attached to the other ends of the link mechanisms 54 (see Figs. 3 and 4).

Each joint part 53 has an end part attached to the attaching base part 52 as an end part 53a, and an end part attached to the corresponding link mechanism 54 as an end part 53b.

The spatial position of the support part 55 corresponds to the position of the surgical instrument 24. In other words, the surgeon can remotely move the position of the surgical instrument 24 by moving the position of the support part 55.

For example, the end part 53a of the joint part 53 is attached to the attaching base part 52 by a ball joint mechanism, and the end part 53a of the joint part 53 is turnable in a direction around a turning axis Ax0. The respective turning axes Ax0 of the end parts 53a of the three joint parts 53 are parallel to the front face part 3a of the base part 3 and are in different directions by 120 degrees.

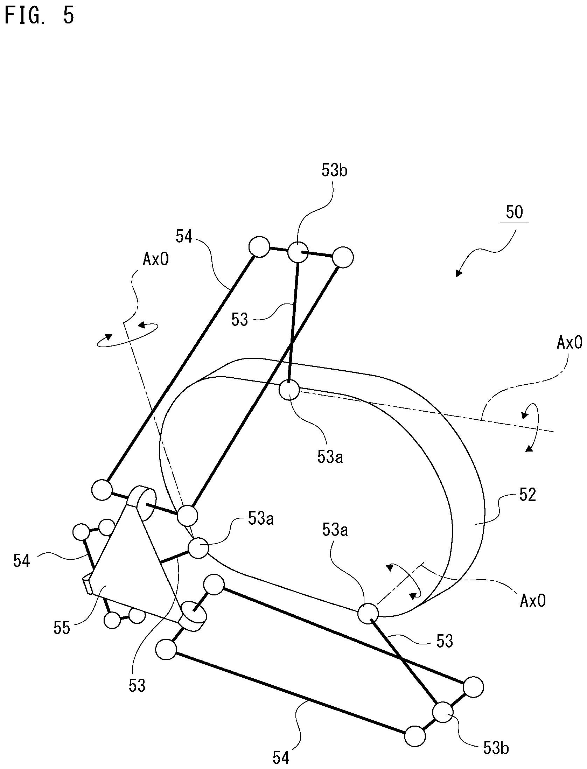

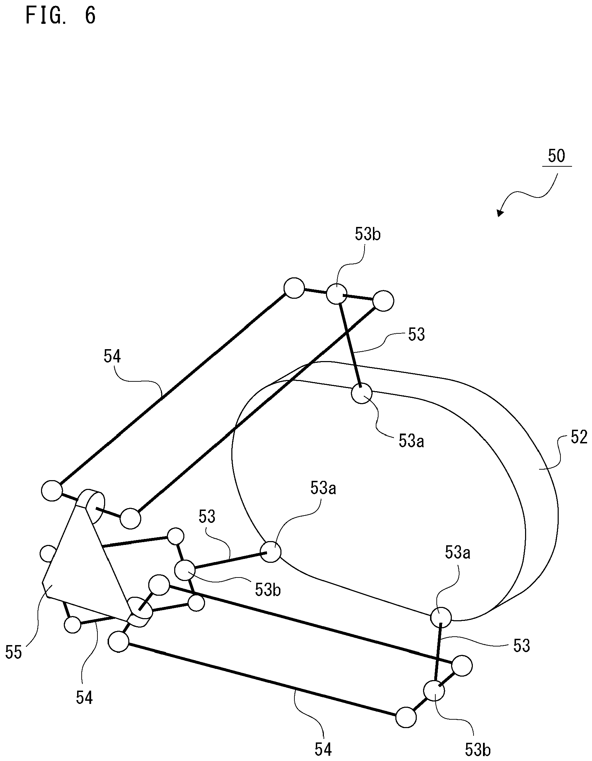

By turning the end part 53a of the joint part 53 in the direction around the turning axis Ax0, the approximate position of the support part 55 in the front and back direction is determined (see Figs. 5 and 6).

The link mechanism 54, one end of which is connected to the end 53b of the joint part 53, includes two parallel links and is deformable into a parallelogram (including a rectangle).

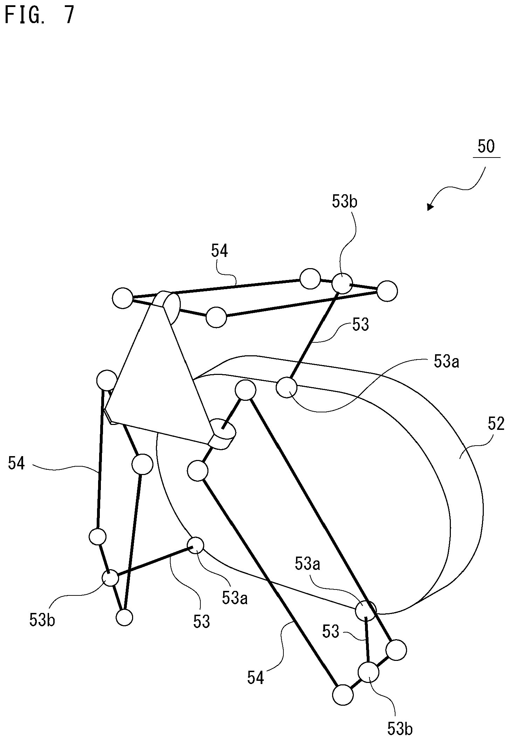

The approximate position of the support part 55 in the up and down and left and right directions is determined by the deformation of the link mechanism 54. For example, by performing a manipulation to move the entire attitude changing mechanism 51 upward from the state illustrated in Fig. 5, the link mechanism 54 is deformed from a substantially rectangle to a parallelogram as illustrated in Fig. 7.

Note that strictly speaking, when the position of the support part 55 in the front and back direction is changed, the angle formed by each joint part 53 and the corresponding link mechanism 54 changes, and the link mechanism 54 is also deformed.

Further, when the support part 55 is moved in the up and down direction or the left and right direction, the link mechanism 54 is deformed, and the end parts 53b of the respective joint parts 53 turn, so that the angle formed by the joint part 53 and the link mechanism 54 changes.

The support part 55 can be translated in the up and down, right and left, front and back directions in space while maintaining the same attitude (orientation) by the movement of the delta mechanism 50.

The configuration and motion of the delta mechanism 50 are well-known techniques, further detailed description will be omitted.

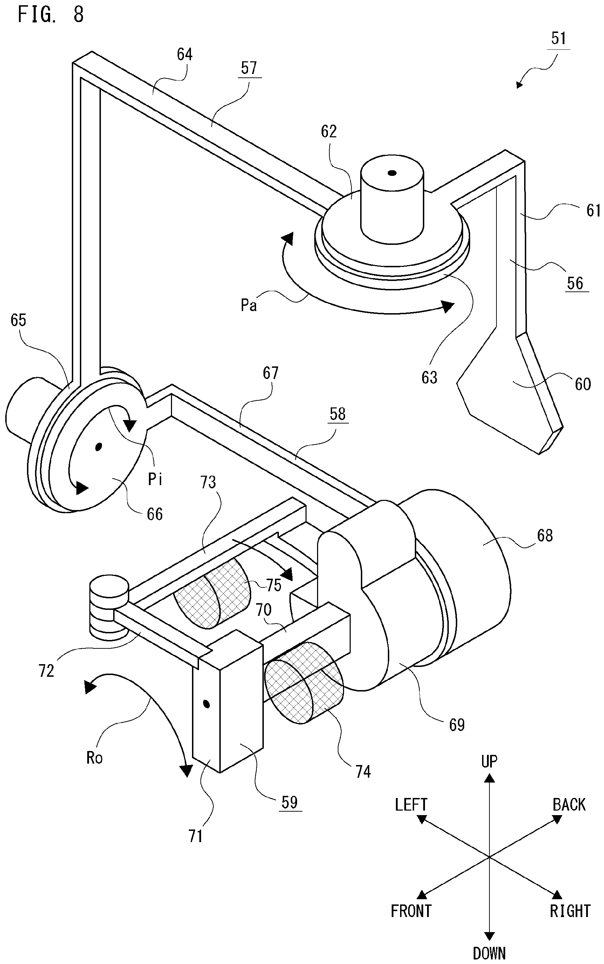

Next, a specific configuration of the attitude changing mechanism 51 supported by the delta mechanism 50 by being attached to the support part 55 of the delta mechanism 50 will be described. Note that Fig. 8 illustrates a schematic configuration of the attitude changing mechanism 51.

The attitude changing mechanism 51 includes a first member 56, a second member 57 that is coupled to the first member 56, a third member 58 that is coupled to the second member 57, and a grasping mechanism 59 that is coupled to the third member 58.

The first member 56 includes a supported part 60 that is supported by the support part 55 and is formed in a plate shape facing front and back, a first connecting part 61 that extends from one end part of the supported part 60 of the supported part 60, and a first turning support part 62 that is continuous with the other end part of the first connecting part 61 and is formed in a disc shape facing up and down.

The second member 57 includes a first turning supported part 63 that is attached to the bottom face part of the first turning support part 62, a second connecting part 64 that is formed in an L-shape extending from the first turning supported part 63 in one of the left and right directions and extending downward from approximately the center part, and a second turning support part 65 that is continuous with the second connecting part 64 and is formed in a disc shape facing left and right.

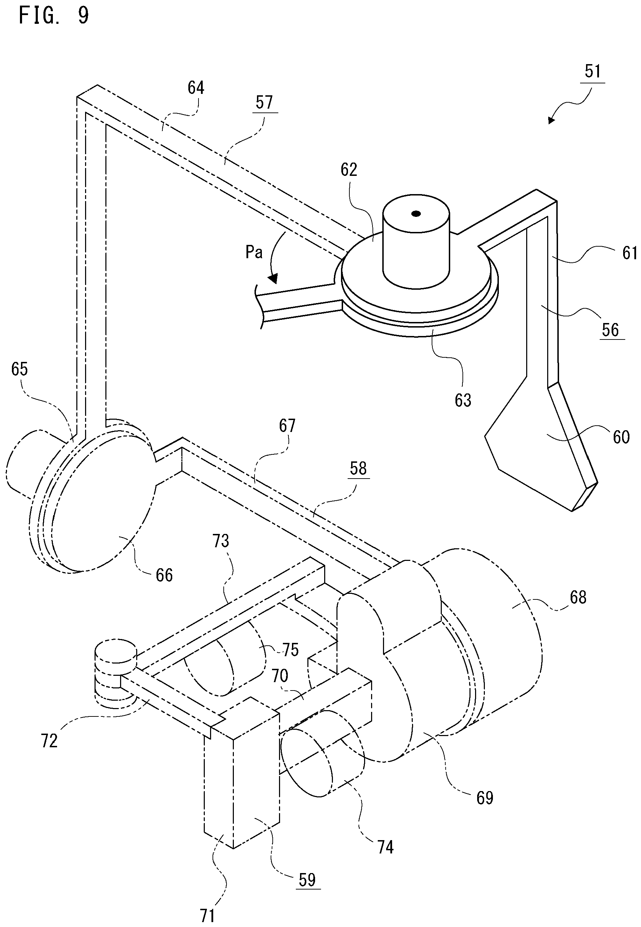

The first turning supported part 63 of the second member 57 is turnable in a direction around an axis extending in the up and down direction with respect to the first turning support part 62 of the first member 56 (see Figs. 8 and 9). This allows the distal end part of the surgical instrument 24L provided on the slave-side device 2 in correspondence with the left manipulation unit 4L to swing left and right. This manipulation is referred to as a pan direction turning manipulation Pa. Note that the left and right direction of the distal end part here refers to the left and right direction when the surgical instrument 24L is in the reference attitude, as described above, and depending on the attitude of the surgical instrument 24L, the left and right direction of the distal end part may also be the vertical direction or an oblique direction.

The third member 58 includes a second turning supported part 66 that is attached to the right side face part of the second turning support part 65 and is formed in a disc shape facing left and right, a third connecting part 67 that extends from one end of the second turning supported part 66 to one of the left and right sides, and an attached end part 68 that is continuous with the other end of the third connecting part 67 and is formed in a disc shape facing in the front and back directions and to which the grasping mechanism 59 is attached.

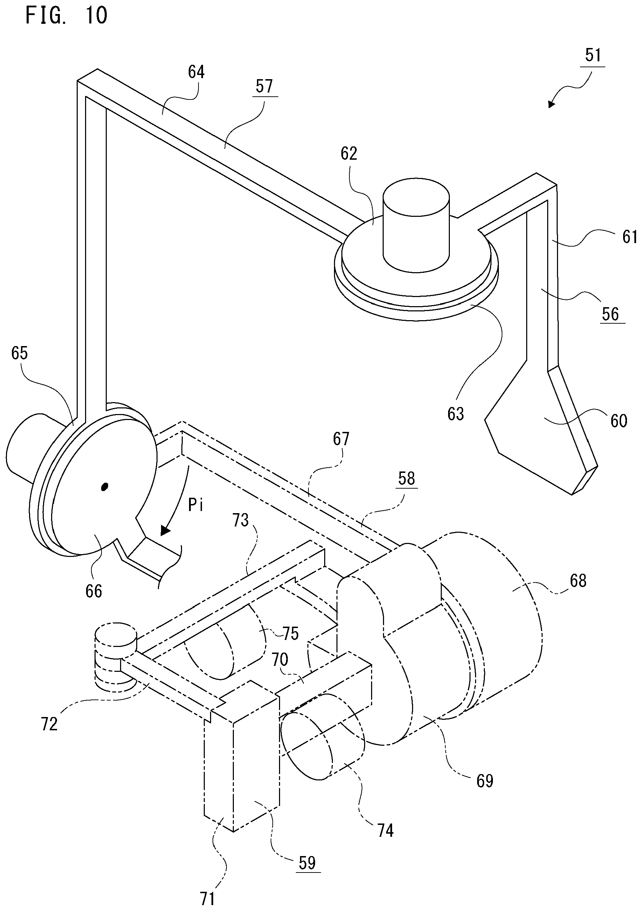

The second turning supported part 66 of the third member 58 is turnable in a direction around an axis extending in the left and right direction with respect to the second turning support part 65 of the second member 57 (see Figs. 8 and 10). This allows the distal end part of the surgical instrument 24L to swing up and down. This manipulation is referred to as a pitch direction turning manipulation Pi.

The grasping mechanism 59 includes an attaching end part 69 that is attached to the attached end part 68 and is formed in a substantially disc shape, a first coupling part 70 that has one end coupled to the front face of the attaching end part 69, a grasping part 71 that is attached to the other end part of the first coupling part 70, a second coupling part 72 that extends laterally from the grasping part 71, and an opening/closing part 73 that is coupled to the end part of the second coupling part 72 opposite to the grasping part 71 and is formed in a substantially L-shape extending forward.

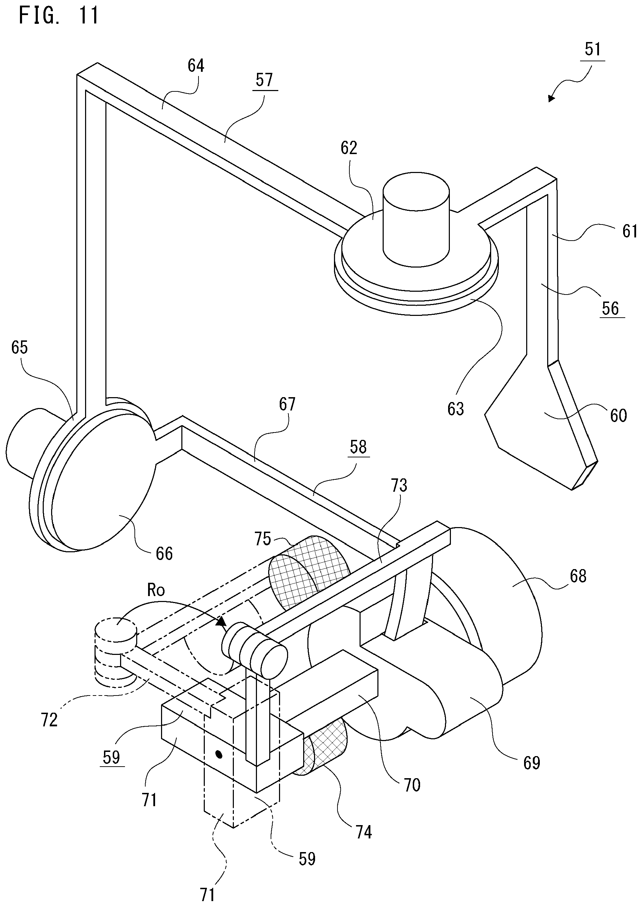

The attaching end part 69 of the grasping mechanism 59 is turnable in a direction around an axis extending in the front- and back direction with respect to the attached end part 68 of the third member 58 (see Figs. 8 and 11). This allows the surgical instrument 24L to turn in a direction around the longitudinal axis of the surgical instrument 24L. This manipulation is referred to as a roll direction turning manipulation Ro.

Note that the directions of the attitude changing mechanism 51 illustrated for the explanation of the pan direction turning manipulation Pa, the pitch direction turning manipulation Pi, and the roll direction turning manipulation Ro are only those in which the attitude changing mechanism 51 is in the reference attitude illustrated in Fig. 8. Therefore, this does not apply when the attitude of the attitude changing mechanism 51 becomes a different attitude from the attitude illustrated in Fig. 8 due to turning of each part. For example, when the first turning supported part 63 of the second member 57 is turned by 90 degrees counterclockwise when viewed from above with respect to the first turning support part 62 of the first member 56, the turning axis of the attaching end part 69 of the grasping mechanism 59 relative to the attaching end part 68 of the third member 58 is an axis extending in the left and right direction rather than in the front and back direction.

The grasping mechanism 59 includes a first grasping aid 74 that is attached to the side face of the first coupling part 70 and into which a finger of the surgeon is inserted, and a second grasping aid 75 that is attached to the bottom face of the opening/closing part 73 and into which another finger of the surgeon is inserted.

For example, the surgeon can manipulate the attitude changing mechanism 51 suitably by inserting the thumb of the left hand into the first grasping aid 74 and inserting the index finger of the left hand into the second grasping aid 75 while grasping the grasping part 71 with the palm of the left hand.

Further, by moving the fingertips of the thumb and index finger of the left hand closer to each other, the distal end part of the opening/closing part 73 is manipulated to approach the attaching end part 69 (closing manipulation Cl). Then, by moving the fingertips of the thumb and index finger of the left hand away from each other, the distal end part of the opening/closing part 73 is manipulated to move away from the attaching end part 69 (opening manipulation Op).

By performing the opening manipulation Op and the closing manipulation Cl, the distal end of the forceps as the surgical instrument 24L provided correspondingly in the slave-side device 2 can be closed or opened.

In this way, the attitude changing mechanism 51 is configured to have four degrees of freedom, and this allows the pan direction turning manipulation Pa to swing the distal end part of the surgical instrument 24 in the pan direction, the pitch direction turning manipulation Pi to swing the distal end part in the pitch direction, the roll direction turning manipulation Ro to turn the distal end part in the roll direction, and an opening/closing manipulation OC to open and close the distal end part of the forceps or the like.

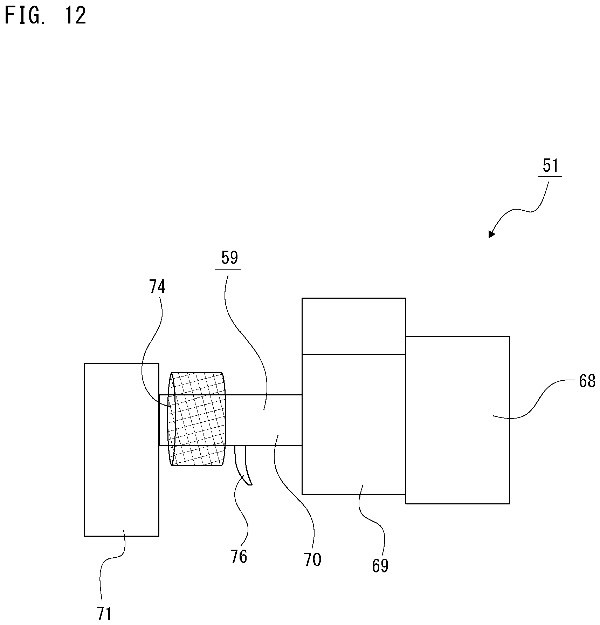

The grasping mechanism 59 includes a clutch manipulator 76 in addition to the above-described parts. As illustrated in Fig. 12, the clutch manipulator 76 is attached to the bottom face part of the first coupling part 70 of the grasping mechanism 59 when the attitude changing mechanism 51 is in the reference attitude, and is, for example, a manipulator formed in a trigger shape.

The clutch manipulator 76 is provided to remove the linkage between the manipulation units 4 of the master-side device 1 and the arms 22 and surgical instruments 24 of the slave-side device 2.

Specifically, when the clutch manipulator 76 is pulled forward (in the direction away from the attaching end part 69, that is, toward the surgeon), even when a manipulation is performed on the manipulation unit(s) 4, each part/unit of the slave-side device 2 is disabled.

A specific purpose of manipulating the clutch manipulator 76 is to remove the linkage between the master-side device 1 and the slave-side device 2 by the surgeon pulling the clutch manipulator 76 toward the surgeon, for example, when any manipulation is difficult because the hand of the surgeon who is grasping the grasping part 71 of the grasping mechanism 59 moves away from the body. Then, while the clutch manipulator 76 is pulled toward the surgeon, the grasping part 71 is translated so as to move the entire attitude changing mechanism 51, and then the manipulation on the clutch manipulator 76 is canceled. As a result, the linkage between the master-side device 1 and the slave-side device 2 is resumed, and the surgeon can resume the surgery with the hand moved to a position where the surgeon is easy to manipulate the manipulation unit 4.

As illustrated in Fig. 12, the surgeon grasps the grasping part 71 with the palm of the right hand with the thumb of the right hand inserted into the first grasping aid 74 and the index finger inserted into the second grasping aid 75, and in that state, the surgeon can pull the clutch manipulator 76 toward the surgeon (the surgeon side) with the middle finger or another finger. Thus, the clutch manipulator 76 can be manipulated without changing the position of the hand that can translate or turn the distal end part of the surgical instrument 24. This improves operability.

Note that in the present embodiment, the position of the grasping mechanism 59 of the master-side device 1 is freely changed while the clutch manipulator 76 is being manipulated with the linkage between the master-side device 1 and the slave-side device 2 removed, but brakes are applied to each part to disable the pan direction turning manipulation Pa, the pitch direction turning manipulation Pi, and the roll direction turning manipulation Ro, that is, to prevent the positional relationship between the first member 56, the second member 57, the third member 58, and the grasping mechanism 59 from changing.

In order to perform the roll direction turning manipulation Ro while the clutch manipulator 76 is being manipulated, a roll clutch function described later may be used. Note that the surgery assisting system S may include a clutch function for the pan direction turning manipulation Pa and the pitch direction turning manipulation Pi.

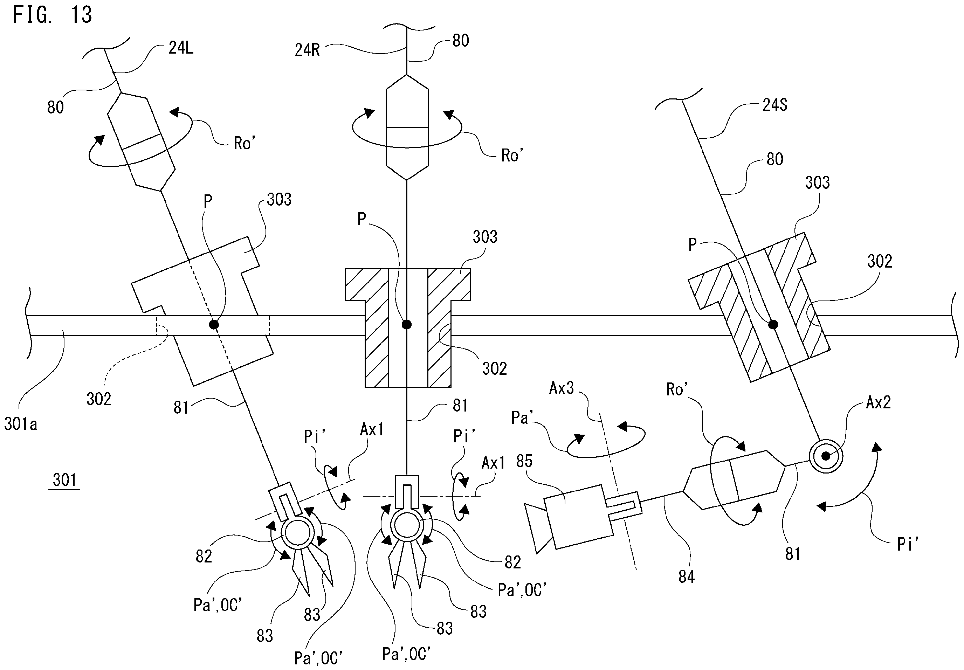

A specific mechanism of the surgical instrument 24 whose attitude changes according to the attitude changing mechanism 51 is schematically illustrated in Fig. 13.

Fig. 13 illustrates as the surgical instruments 24 the surgical instruments 24R, 24L, and the endoscope 24S.

The surgical instruments 24R and 24L are each formed into a shaft shape as a whole, for example, and are configured to include a first shaft part 80 that is held by the surgical instrument holding device 23, a second shaft part 81 that has one end part connected to the distal end part of the first shaft part 80, a turning coupling part 82 that is held at the other end part of the second shaft part 81, and two distal end pieces 83 that are supported by the turning coupling part 82.

The second shaft part 81 is turnable in a direction around the axis of the second shaft part 81 with respect to the first shaft part 80, and turns in response to the roll direction turning manipulation Ro on the grasping mechanism 59 (turning motion Ro').

The turning coupling part 82 is turnable in a direction around a first axis Ax1 illustrated in Fig. 13 with respect to the second shaft part 81, and turns in response to the pitch direction turning manipulation Pi on the grasping mechanism 59 (turning motion Pi').

Each of the two distal end pieces 83 is turnable with respect to the turning coupling part 82 in a direction around an axis perpendicular to each of the turning axis of the turning motion Ro' and the turning axis of the turning motion Pi'.

In response to the pan direction turning manipulation Pa on the grasping mechanism 59, the two distal end pieces 83 turn in the same direction (turning motion Pa'). Further, in response to the opening manipulation Op of the opening/closing manipulation OC on the grasping mechanism 59, the two distal end pieces 83 turn in a direction away from each other (opening motion Op'). Furthermore, in response to the closing manipulation Cl of the opening/closing manipulation OC on the grasping mechanism 59, the two distal end pieces 83 turn in a direction toward each other (closing motion Cl').

In this way, the turning motion Pa' and an opening/closing motion OC' of the distal end pieces 83 are achieved.

Next, a configuration example of the endoscope 24S will be described.

The endoscope 24S is formed into a shaft shape as a whole, for example, and is configured to include a first shaft part 80 that is held by the surgical instrument holding device 23, a second shaft part 81 that has one end part connected to the distal end part of the first shaft part 80, a third shaft part 84 that is connected to the other end part of the second shaft part 81, and a camera head 85 that is held by the third shaft part 84.

In the above-described example, a configuration has been described in which the position and attitude of the surgical instrument 24R change in response to a manipulation on the right manipulation unit 4R, and the position and attitude of the surgical instrument 24L change in response to a manipulation on the left manipulation unit 4L.

However, during surgery, there are situations in which it is necessary to change the viewing angle of the endoscope 24S. In that case, the position and attitude of the endoscope 24S may be changed according to a manipulation on either the right manipulation unit 4R or the left manipulation unit 4L. For example, the linkage between the right manipulation unit 4R and the endoscope 24S may be started after the linkage between the right manipulation unit 4R and the surgical instrument 24R is removed.

This makes it possible to obtain an image based on an appropriate viewing angle from the endoscope 24S, making it easier to perform surgery.

Specifically, the second shaft part 81 of the endoscope 24S is turnable in a direction around a second axis Ax2 illustrated in Fig. 13 with respect to the first shaft part 80, and turns in response to the pitch direction turning manipulation Pi on the grasping mechanism 59 (turning motion Pi').

The third shaft part 84 of the endoscope 24S is turnable in a direction around the axis of the third shaft part 84 with respect to the second shaft part 81, and turns in response to the roll direction turning manipulation Ro on the grasping mechanism 59 (turning motion Ro').

The camera head 85 is turnable in a direction around a third axis Ax3 illustrated in Fig. 13 with respect to the third shaft part 84, and turns in response to the pan direction turning manipulation Pa on the grasping mechanism 59 (turning motion Pa').

In this way, the change of the attitude of the endoscope 24S is achieved.

Note that although detailed description is omitted, to insert the distal end part of the surgical instrument 24 into the body cavity 301, a trocar 303 placed in the corresponding port 302 formed in the abdominal wall 301a is used. To change the position and attitude of the surgical instrument 24, a pivot point P is set inside the trocar 303, that is, near the center of the port 302.

When the distal end part of the surgical instrument 24 is inserted into the body cavity 301 of the patient 300, the position of the surgical instrument 24 is controlled so that a part of the surgical instrument 24 always passes through the pivot point P, preventing a load on the tissue near the body surface of the patient 300 from occurring before and after the change of the position and attitude of the surgical instrument 24 and thus making it possible to ensure safety.

### <3. Functional Configuration>

The functional configuration of the surgery assisting system S to smoothly perform surgery will be described.

### <3-1. Functional Configuration of Master-side Device>

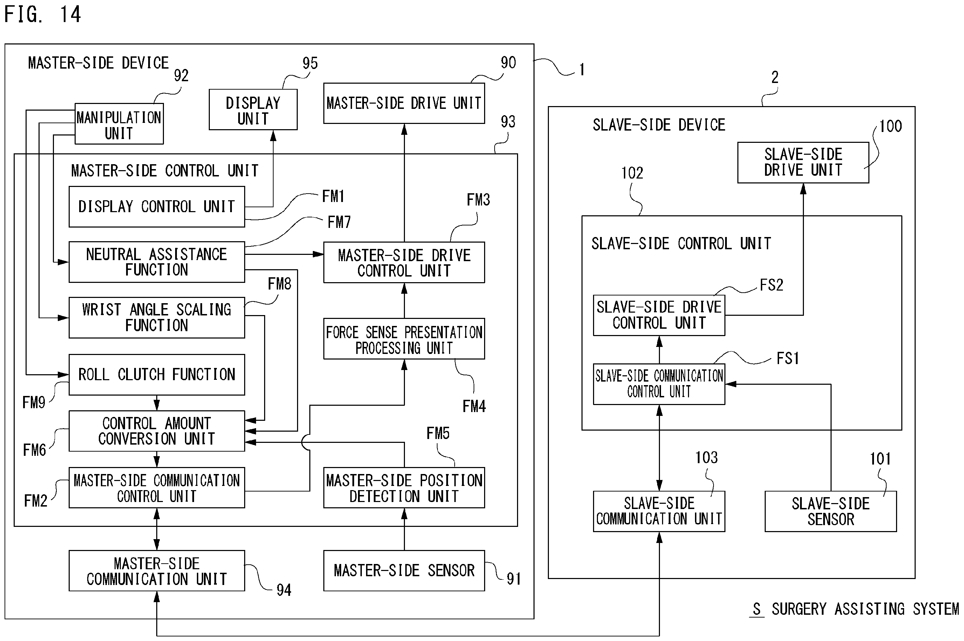

The functional configuration of the master-side device 1 will be described first with reference to Fig. 14. This figure is a functional block diagram of the master-side device 1 and the slave-side device 2 in the surgery assisting system S.

The master-side device 1 includes a master-side drive unit 90, a master-side sensor 91, a manipulation unit 92, a master-side control unit 93, a master-side communication unit 94, and a display unit 95.

The master-side drive unit 90 is provided as each part of the delta mechanism 50 and each part of the attitude changing mechanism 51 in the manipulation unit 4. Specifically, the master-side drive unit 90 is an actuator for turning the end part 53a of the joint part 53 in a direction around the turning axis Ax0 illustrated in Fig. 5, an actuator to control a relative turning state between the link mechanism 54 and the support part 55, or an actuator for turning the second member 57 in the direction of the pan direction turning manipulation Pa with respect to the first member 56 illustrated in Fig. 8.

The master-side drive unit 90 is driven based on a control signal supplied from the master-side control unit 93 to change the position and attitude of each unit of the master-side device 1.

The master-side sensor 91 is provided as a sensor that senses the positional relationship of the parts, and detects, for example, a turning angle representing the turning state of the end part 53a of the joint part 53 in the direction around the turning axis Ax0 illustrated in Fig. 5, a turning angle representing the turning state of the second member 57 with respect to the first member 56 illustrated in Fig. 8, and the like to output the resulting detection signal.

The manipulation unit 92 corresponds to, for example, the clutch manipulator 76, the first foot button 5, the second foot button 6, and the like described above. Further, if the first monitor 7, the second monitor 8, and the like each have a touch panel function, a touch panel portion is also an example of the manipulation unit 92. Note that if a mouse or the like for performing an operation on a menu screen or the like displayed on the second monitor 8 or the like is connected, the mouse or the like is also the manipulation unit 92.

In addition, if the master-side device 1 supports voice input, a microphone or the like included in the master-side device 1 may be the manipulation unit 92.

The display unit 95 corresponds to the first monitor 7, the second monitor 8, and the like described above.

The master-side control unit 93 is configured to include a central processing unit (CPU), a read only memory (ROM), a random access memory (RAM), and others, and implements a predetermined function by an arithmetic processing unit such as the CPU executing a program stored in the ROM or a program loaded into the RAM.

Specifically, the master-side control unit 93 includes a display control unit FM1, a master-side communication control unit FM2, a master-side drive control unit FM3, a force sense presentation processing unit FM4, a master-side position detection unit FM5, a control amount conversion unit FM6, a neutral assistance function FM7, a wrist angle scaling function FM8, and a roll clutch function FM9.

The display control unit FM1 performs display control on the first monitor 7 and the second monitor 8. In the present embodiment, the display control unit FM1 causes the second monitor 8 to display setting screens for various settings. On each setting screen, a drop-down list, buttons, and the like are arranged each serving as the manipulation unit 92 as appropriate, and manipulation information for the manipulation unit 92 is notified to the neutral assistance function FM7, the wrist angle scaling function FM8, and the roll clutch function FM9.

Further, the display control unit FM1 receives image data captured by the endoscope 24S serving as the surgical instrument 24 included in the slave-side device 2 through the master-side communication unit 94, and performs processing of outputting it to the first monitor 7.

The master-side communication control unit FM2 performs processing for transmitting and receiving various types of information to and from the slave-side device 2 using the master-side communication unit 94.

The master-side drive control unit FM3 controls various actuators each provided as the master-side drive unit 90 in order to control each part/unit of the master-side device 1.

Note that the master-side drive control unit FM3 may be controlled by other functions or parts/units in order to implement various functions of the master-side control unit 93. In other words, the master-side drive control unit FM3 is capable of executing processing for controlling the master-side drive unit 90 based on commands output from other functions or parts/units.

One example is force sense presentation control. Specifically, the force sense presentation processing unit FM4 performs processing for presenting to the surgeon a force sense as if the surgeon were handling the surgical instrument 24 with the surgeon's hand, in accordance with the movement of the surgical instrument 24 such as forceps included in the slave-side device 2. For example, when the surgical instrument 24 hits an obstacle, the master-side drive unit 90 is driven in a direction in which the manipulation input by the surgeon is canceled, making the force necessary to move the surgical instrument 24 larger than usual, so that the surgeon can sense that the surgical instrument 24 hits the obstacle.

In order to perform such a control, the force sense presentation processing unit FM4 instructs the master-side drive control unit FM3.

The master-side control unit 93 performs a control to drive each part/unit of the slave-side device 2 in accordance with a surgeon's manipulation on the master-side device 1.

Specifically, the master-side position detection unit FM5 detects the position, attitude, and the like of the master-side drive unit 90 based on the output signal from the master-side sensor 91, and outputs the result of detection to the control amount conversion unit FM6.

The control amount conversion unit FM6 calculates the amount of change in the master-side drive unit 90 from the position, attitude, and the like of the master-side drive unit 90 input from the master-side position detection unit FM5. The control amount conversion unit FM6 converts the amount of change into an amount of control for each drive unit of the slave-side device 2 based on the amount of change. Note that the position and attitude of each surgical instrument 24 of the slave-side device 2 are changed so that the surgical instrument 24 passes through the pivot point P set near the corresponding port 302 of the patient 300. The control amount conversion unit FM6 performs processing of converting the translational movement of the support part 55 of the master-side device 1 into a turning motion or other type of motion of the drive unit of the slave-side device 2 so that the surgical instrument 24 passes through the pivot point P.

The amount of control (amount of drive) calculated for each drive unit of the slave-side device 2 by the control amount conversion unit FM6 is transmitted from the master-side communication unit 94 to the slave-side device 2 through processing performed by the master-side communication control unit FM2.

The neutral assistance function FM7, the wrist angle scaling function FM8, and the roll clutch function FM9 can be regarded as auxiliary functions for performing surgery smoothly.

### <3-1-1. Neutral Assistance Function>

The neutral assistance function FM7 is a function for guiding the delta mechanism 50 of the manipulation unit 4 included in the master-side device 1 to an appropriate position.

For example, the neutral assistance function FM7 is a function that is started in response to simultaneous manipulation (manipulation of pulling toward the surgeon) of the clutch manipulator 76 provided in the attitude changing mechanism 51 of the left manipulation unit 4L and the clutch manipulator 76 provided in the attitude changing mechanism 51 of the right manipulation unit 4R. Note that this way of manipulation is just an example, and the function may be executed by voice operation, or, for example, the function may be executed by pressing the first foot button 5 and the second foot button 6 at the same time. Furthermore, the function may be executed by inputting a predetermined manipulation pattern to the clutch manipulator 76 or the like.

The neutral assistance function FM7 is a function that moves only the delta mechanism 50 of the master-side device 1 to a predetermined position without moving each part/unit of the slave-side device 2. Therefore, to execute the neutral assistance function FM7, the control amount conversion unit FM6 is notified that the linkage with the slave-side device 2 is to be removed (suspended). In response to this, the control amount conversion unit FM6 calculates as "0" an amount of motion of the turning motion of the drive unit of the slave-side device 2 and other types of motion, regardless of the result of detection by the master-side sensor 91.

The neutral assistance function FM7 performs processing for guiding the delta mechanism 50 to an appropriate position in a state where the linkage with the slave-side device 2 is terminated.

Note that while only software may be used to terminate the linkage between the master-side device 1 and the slave-side device 2 as described above, a hardware mechanism may be used.

For example, each movable part of the slave-side device 2 may have an electromagnetic brake mechanism as a non-excitation type of brake that is configured to apply a brake and maintain the position and attitude when the power supply is cut off. In this case, the power supply to the electromagnetic brake is stopped to apply a brake to each movable part, thereby maintaining the position and attitude of each movable part. This can be achieved without any modification to the calculation of the amount of motion by the control amount conversion unit FM6.

The delta mechanism 50 of the master-side device 1 has a predetermined range of movement in the up and down direction, left and right direction, and front and back direction, which limits the possible range of the position of the attitude changing mechanism 51 attached to the delta mechanism 50 (or the position of the grasping mechanism 59) in each direction.

For example, if the surgeon performs a manipulation near the end of the range of movement of the grasping mechanism 59, the range of movement of the delta mechanism 50 will reach its limit, making it impossible to perform the intended manipulation, which may fail to provide adequate treatment to the patient 300.

In order to deal with such a situation, the delta mechanism 50 is guided by the neutral assistance function FM7.

Specifically, the neutral assistance function FM7 moves each part of the left and right delta mechanisms 50 so that the left and right grasping mechanisms 59 are each located approximately at the center of the range of movement in each direction.

As a result, the grasping mechanism 59 grasped by the surgeon can resume the surgery with a certain range of movement remaining on either side in each of the up and down direction, left and right direction, and front and back direction, and thus, adequate treatment can be provided.

For this purpose, the neutral assistance function FM7 instructs the master-side drive control unit FM3.

Note that, in order to guide the grasping mechanism 59 to a predetermined position, force sense presentation may be performed to convey the direction in which the palm of the surgeon who is grasping the grasping mechanism 59 should be moved.

After the grasping mechanism 59 is moved to the predetermined position by the neutral assistance function FM7, the linkage between the master-side device 1 and the slave-side device 2 can be resumed. The linkage may be resumed automatically, or may be configured not to be resumed unless a manual manipulation is performed.

Note that when the clutch manipulator 76 is pulled toward the surgeon, the linkage between the master-side device 1 and the slave-side device 2 is terminated, and when the clutch manipulator 76 is released from being pulled toward the surgeon, the linkage between the master-side device 1 and the slave-side device 2 is resumed. However, when the left and right clutch manipulators 76 are manipulated simultaneously, even when the left and right clutch manipulators 76 are released from being pulled toward the surgeon during automatic control of the grasping mechanism 59 by the neutral assistance function FM7, it is preferable from the viewpoint of safety that the linkage between the master-side device 1 and the slave-side device 2 is not resumed unless the automatic control is completed.

Further, when the left and right clutch manipulators 76 are released from being pulled toward the surgeon during automatic control of the grasping mechanism 59 by the neutral assistance function FM7, the automatic control by the neutral assistance function FM7 may be canceled to immediately resume the linkage between the master-side device 1 and the slave-side device 2.

In the present embodiment, it is possible to switch on/off of the neutral assistance function FM7. This switching is realized by performing an operation on a setting screen displayed on the second monitor 8.

An example of a setting screen G1 will be described with reference to the attached drawings.

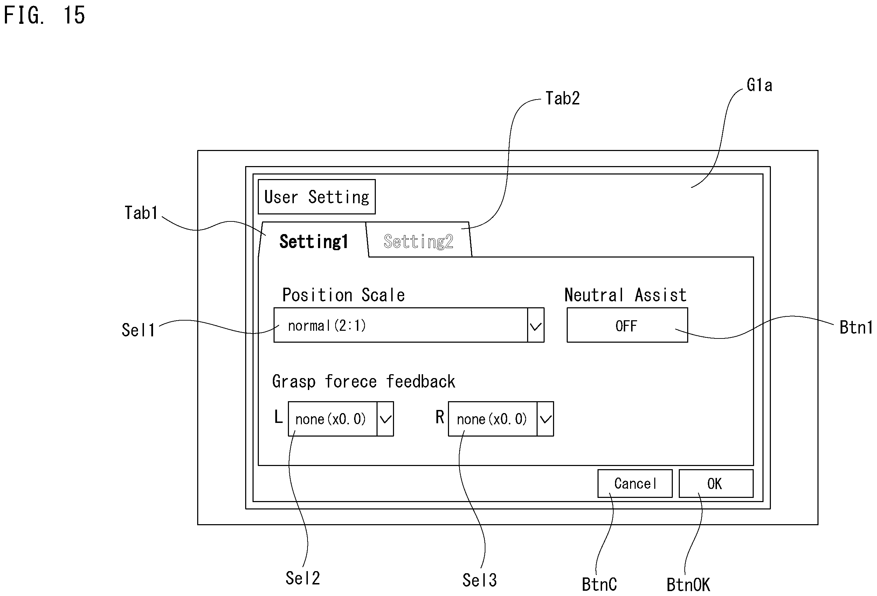

The setting screen G1 is configured as a screen with two tabs, and Fig. 15 illustrates a state in which a first setting tab Tab1 displayed as "Setting1" is selected. This screen is referred to as a setting screen G1a.

The setting screen G1a includes a position scaling selection field Sel1, a neutral assistance button Btn1, a left force sense presentation strength selection field Sel2, a right force sense presentation strength selection field Sel3, a cancel button BtnC, and an execution button BtnOK.

Among these, a control for switching on/off of the neutral assistance function FM7 is the neutral assistance button Btn1.

The current state is displayed on the neutral assistance button Btn1, and on and off are switched by operating the neutral assistance button Btn1.

By operating the cancel button BtnC, the setting screen G1 can be closed without reflecting the settings set on the setting screen G1a.

Further, by operating the execution button BtnOK, the settings screen G1 can be closed with the settings set on the settings screen G1a reflected.

### <3-1-2. Wrist Angle Scaling Function>

The wrist angle scaling function FM8 is a function for changing the amount of turning of the turning motion Pa' for the pan direction turning manipulation Pa, the amount of turning of the turning motion Pi' for the pitch direction turning manipulation Pi, and the amount of turning of the turning motion Ro' for the roll direction turning manipulation Ro.

The possible amount of movement of the human wrist is approximately fixed, and for example, it is difficult to rotate the wrist by 360 degrees while performing precise manipulations. The wrist angle scaling function FM8 is a function to solve such a problem.

The ratio between the amount of manipulation and the amount of turning can be achieved by manipulating the manipulation unit 92. The wrist angle scaling function FM8 notifies the control amount conversion unit FM6 of the ratio set based on the manipulation of the manipulation unit 92. Based on that notification, the control amount conversion unit FM6 obtains the final amount of motion by multiplying the amount of manipulation of each part of the slave-side device 2 calculated from the amount of manipulation by the above-mentioned ratio.

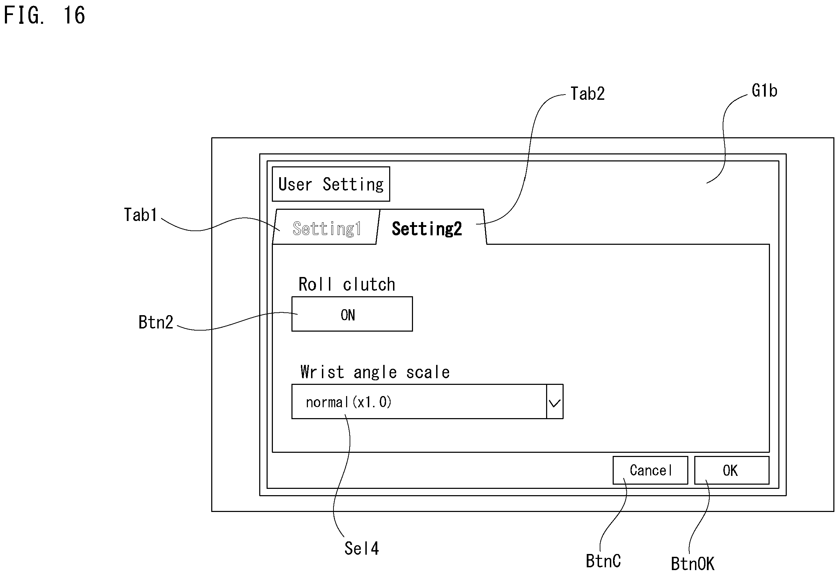

The manipulation unit 92 for determining the ratio between the amount of manipulation and the amount of turning may be a mechanical knob such as a dial knob, or may be a control displayed on a screen realized by software. An example is illustrated in Fig. 16.

Fig. 16 illustrates a state in which a second setting tab Tab2 displayed as "Setting2" is selected on the setting screen G1 with two tabs. This screen is referred to as a setting screen G1b.

A roll clutch button Btn2, a ratio selection field Sel4, a cancel button BtnC, and an execution button BtnOK are arranged on the setting screen G1b.

Among these, a control for determining the ratio between the amount of manipulation and the amount of turning in the wrist angle scaling function FM8 is the ratio selection field Sel4.

The ratio selection field Sel4 allows a selection from three options: "1.0", "1.5", and "2.0". For example, for a selection of "2.0", when the pan direction turning manipulation Pa is performed by 30 degrees in the master-side device 1, the distal end part of the surgical instrument 24 in the slave-side device 2 is controlled to turn by 60 degrees.

Note that the options to be selected in the ratio selection field Sel4 may include a value less than 1.0. This makes it suitable for performing precise work.

Further, a slider control may be provided instead of the ratio selection field Sel4 so that the ratio can be changed almost steplessly.

The operations on the cancel button BtnC and the execution button BtnOK are the same as those on the setting screen G1b, and thus, the description thereof will be omitted.

Note that the master-side control unit 93 may have a scaling function for adjusting the amount of turning of the turning motion Pa' for the pan direction turning manipulation Pa, a scaling function for adjusting the amount of turning of the turning motion Pi' for the pitch direction turning manipulation Pi, and a scaling function for adjusting the amount of turning of the turning motion Ro' for the roll direction turning manipulation Ro. In addition, these scaling functions may be set with different ratios.

Further, the master-side control unit 93 may not have one or some of the scaling functions.

### <3-1-3. Roll Clutch Function>

The roll clutch function FM9 is a function that determines whether or not to perform the turning motion Ro' of the distal end part of the surgical instrument 24 of the slave-side device 2 in response to the roll direction turning manipulation Ro.

This function aims to solve the problem of difficulty in performing surgery due to the range of movement of the human wrist.

For example, when a turning manipulation of 180 degrees is performed as the roll direction turning manipulation Ro, specifically, when the wrist turns so that the palm turns from facing down to facing up, the distal end part of the surgical instrument 24 performs the turning motion Ro' of 180 degrees accordingly. In this state, if it is desired to perform the turning motion Ro' in the same direction, it may be difficult due to the range of movement of the surgeon's wrist. In that case, by the roll clutch function FM9, the state is changed to such a state that the turning motion Ro' of the distal end part of the surgical instrument 24 is not performed even when the roll direction turning manipulation Ro is performed.

Next, the surgeon turns the wrist so that the palm faces down again, and then, by the roll clutch function FM9, the state is changed to such a state that the roll direction turning motion Ro' of the distal end part of the surgical instrument 24 is performed again.

Repeating this process makes it possible to perform the turning motions Ro' in the same direction any number of times.

The manipulation unit 92 for turning on/off the linkage between the roll direction turning manipulation Ro and the turning motion Ro' may be a mechanical manipulator such as a button, or may be a control displayed on a screen realized by software.

In the example illustrated in Fig. 16, the roll clutch button Btn2 on the setting screen G1b is a control for the roll clutch function FM9.

The current state is displayed on the roll clutch button Btn2, and by operating the roll clutch button Btn2, the linkage between the manipulation in the roll direction and the turning motion can be switched on/off.

### <3-2. Functional Configuration of Slave-side Device>

The slave-side device 2 includes a slave-side drive unit 100, a slave-side sensor 101, a slave-side control unit 102, and a slave-side communication section 103.

The slave-side drive unit 100 is provided as a movable part in each part of the arm 22, each part of the surgical instrument holding device 23, and each part of the surgical instrument 24, which are included in the slave-side device 2.

The slave-side drive unit 100 is driven based on a control signal supplied from the slave-side control unit 102 to perform various translational motions and turning motions, so that the position and attitude of the distal end part of the surgical instrument 24 can be changed.

The slave-side sensor 101 is provided as a sensor that senses the positional relationship of the movable parts of the slave-side device 2, and outputs a detection signal corresponding to, for example, the turning position of the second shaft part 31 with respect to the first shaft part 80 illustrated in Fig. 13, the turning position of the distal end pieces 83 with respect to the turning coupling part 82, and the like.

Note that an image sensor or the like included in the endoscope 24S is also an example of the slave-side sensor 101.

The slave-side control unit 102 is configured to include a CPU, ROM, RAM, and others, and implements a predetermined function by an arithmetic processing unit such as the CPU executing a program stored in the ROM or a program loaded into the RAM.

Specifically, the slave-side control unit 102 includes a slave-side communication control unit FS1 and a slave-side drive control unit FS2.

The slave-side communication control unit FS1 performs processing for transmitting and receiving various types of information to and from the master-side device 1 using the slave-side communication unit 103.

The slave-side drive control unit FS2 controls various actuators each provided as the slave-side drive unit 100 in order to control the drive units provided as parts of the slave-side device 2. Specifically, the slave-side drive control unit FS2 outputs a control signal to each actuator of the slave-side device 2 based on the amount of control (amount of drive) calculated by the control amount conversion unit FM6 of the master-side control unit 93.

### <4. Processing Flow>

An example of the flow of processing will be described that is executed by an arithmetic processing unit serving as the master-side control unit 93 to implement the above-described various functions of the master-side device 1.

### <4-1. Processing Related to Neutral Assistance Function>

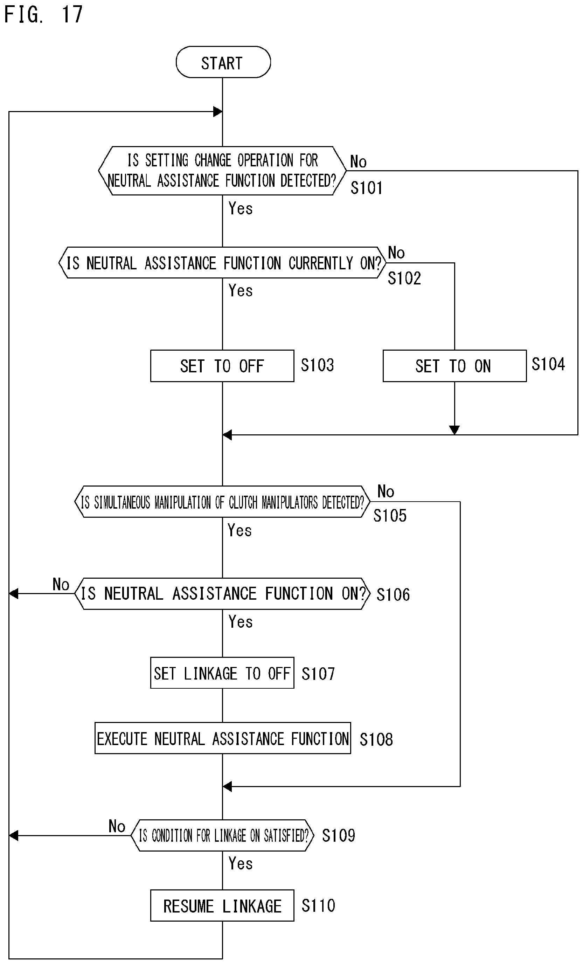

As processing related to the neutral assistance function FM7, the master-side control unit 93 executes a series of processes illustrated in Fig. 17.

In step S101, the master-side control unit 93 determines whether or not a setting change operation for the neutral assistance function has been detected. The setting change operation is, for example, an operation of pressing the neutral assistance button Btn arranged on the setting screen G1a to change the current state, and then pressing the execution button BtnOK.

If it is determined that no setting change operation has been detected, the master-side control unit 93 proceeds to step S105 without executing processes of step S102 to step S104.

On the other hand, if it is determined that a setting change operation has been detected, the master-side control unit 93 determines in step S102 a setting indicating whether or not to start the neutral assistance function in response to a predetermined manipulation input such as simultaneously pressing the two clutch manipulators 76 is on or off.

If the neutral assistance function is on, the master-side control unit 93 sets the function to off in step S103.

On the other hand, if the neutral assistance function is off, the master-side control unit 93 sets the function to on in step S 104.

After executing step S103 or step S104, or after making a "No" determination in step S101, the master-side control unit 93 determines in step S105 whether or not the predetermined manipulation input, that is, the simultaneous manipulation input of the two clutch manipulators 76 has been detected.

If it is determined that the manipulation input has not been detected, the master-side control unit 93 proceeds to step S109.

On the other hand, if it is determined that the manipulation input has been detected, the master-side control unit 93 determines in step S106 whether or not the neutral assistance function is on.

If it is determined that the neutral assistance function is on, the master-side control unit 93 removes (suspends) the linkage between the master-side device 1 and the slave-side device 2 in step S107, and executes the neutral assistance function in step S108.

Specifically, the master-side control unit 93 moves each part of the left and right delta mechanisms 50 so that the left and right grasping mechanisms 59 are each located approximately at the center of the range of movement in each direction.

Note that the neutral assistance function may be executed using the force sense presentation by the force sense presentation processing unit FM4, as described above.

On the other hand, if it is determined that the neutral assistance function is off, the master-side control unit 93 returns to the process of step S101.

After executing step S108, the master-side control unit 93 determines in step S109 whether or not a condition for resuming the linkage between the master-side device 1 and the slave-side device 2 is satisfied.

If it is determined that the condition for resuming the linkage is satisfied, the master-side control unit 93 resumes the linkage between the master-side device 1 and the slave-side device 2 in step S110, and returns to the process of step S101.

On the other hand, if it is determined that the condition for resuming the linkage is not satisfied, the processing returns to step S101 with the linkage between the master-side device 1 and slave-side device 2 being suspended.

Repeatedly executing the series of processes illustrated in Fig. 17 at predetermined time intervals during the surgery assisting system S running allows the surgeon to start the neutral assistance function at any timing. Further, the series of processes illustrated in Fig. 17 ends, for example, when the surgery assisting system S is powered off.

### <4-2. Processing Related to Wrist Angle Scaling Function>

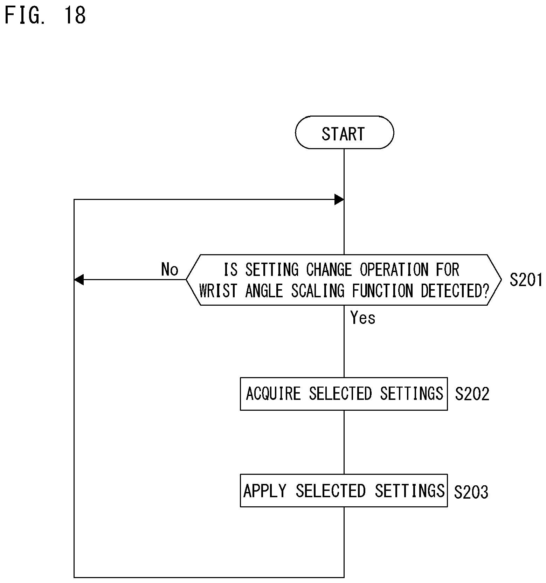

As processing related to the wrist angle scaling function FM8, the master-side control unit 93 executes a series of processes illustrated in Fig. 18.

First, the master-side control unit 93 determines in step S201 whether or not a setting change operation for the wrist angle scaling function has been detected. The setting change operation is, for example, an operation of selecting a different ratio from the current ratio from the ratio selection field Sel4 arranged on the setting screen G1b and then pressing the execution button BtnOK.

If it is determined that no setting change operation has been detected, the master-side control unit 93 executes the process of step S201 again.

On the other hand, if it is determined that a setting change operation has been detected, the master-side control unit 93 acquires the selected settings in step S202, and applies the selected settings in step S203. Applying the selected settings is performed, for example, by notifying the control amount conversion unit FM6 of ratio information. The control amount conversion unit FM6 obtains the amount of motion of each movable part of the slave-side device 2 based on the notified ratio information.

After ending the process of step S203, the master-side control unit 93 returns to the process of step S201.

Repeatedly executing the series of processes illustrated in Fig. 18 at predetermined time intervals during the surgery assisting system S running allows the surgeon to change the ratio between the amount of manipulation and the amount of motion at any timing based on the wrist angle scaling function. Further, the series of processes illustrated in Fig. 18 ends, for example, when the surgery assisting system S is powered off.

### <4-3. Processing Related to Roll Clutch Function>

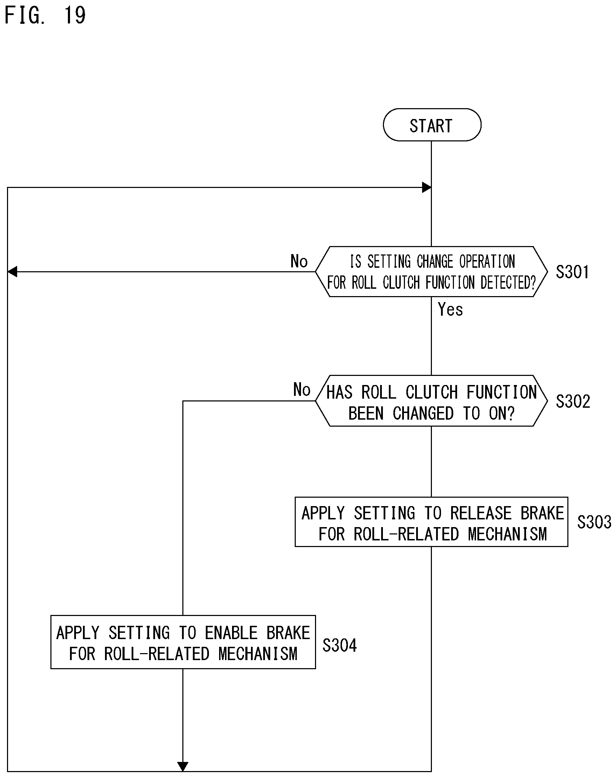

As processing related to the roll clutch function FM9, the master-side control unit 93 executes a series of processes illustrated in Fig. 19.

First, in step S301, the master-side control unit 93 determines whether or not a setting change operation for the roll clutch function has been detected. The setting change operation is, for example, an operation of pressing the roll clutch button Btn2 arranged on the setting screen G1b to change the current state, and then pressing the execution button BtnOK.

If it is determined that no setting change operation has been detected, the master-side control unit 93 executes the process of step S301 again.

On the other hand, if it is determined that a setting change operation has been detected, the master-side control unit 93 determines in step S302 whether or not the roll clutch function has been changed from off to on.

If it is determined that the change from off to on has been made, the master-side control unit 93 applies a setting for releasing the brake of the roll-related mechanism when the clutch manipulator 76 is pulled toward the surgeon in step S303. This allows the surgeon to perform the roll direction turning manipulation Ro in addition to a translational motion of the grasping mechanism 59 while pulling the clutch manipulator 76 toward the surgeon.

For example, for the two distal end pieces 83 of the surgical instrument 24 as forceps having different shapes, there are cases where it is necessary to perform the turning motion Ro' of 180 degrees in order to bring the distal end part of the surgical instrument 24 into a suitable attitude. The roll clutch function is suitable in such cases.

If it is determined in step S302 that the roll clutch function has been changed from on to off, the master-side control unit 93 applies a setting for applying the brake of the roll-related mechanism when the clutch manipulator 76 is pulled toward the surgeon in step S304. As a result, even when the clutch manipulator 76 is pulled toward the surgeon, the brake is applied so that the attaching end part 69 does not turn relative to the attached end part 68 illustrated in Fig. 8.

After ending the process of step S303 or the process of step S304, the master-side control unit 93 returns to the process of step S301.

Repeatedly executing the series of processes illustrated in Fig. 19 at predetermined time intervals during the surgery assisting system S running allows the surgeon to switch the roll clutch function between on and off at any timing. Further, the series of processes illustrated in Fig. 19 ends, for example, when the surgery assisting system S is powered off.

### <5. Modification Example>

The surgery assisting system S may be capable of scaling the amount of manipulation and the actual amount of movement to perform translational movement of the distal end part of the surgical instrument 24. For example, in the setting screen G1a illustrated in Fig. 15, an example is illustrated in which the position scaling selection field Sel1 is arranged.

The position scaling selection field Sel1 allows a selection of ratios between the amount of manipulation for the surgeon to perform a manipulation for translating the grasping mechanism 59 using the delta mechanism 50 and the amount of movement for the distal end part of the surgical instrument 24 to translate in accordance with that manipulation.

The position scaling selection field Sel1 allows a selection from "2:1", "3: 1", "1:1", and others. Providing options for reducing the amount of movement relative to the amount of manipulation makes it possible to suitably respond to delicate surgical operations.

By the above-described neutral assistance function FM7, processing has been described that is performed for guiding the grasping mechanism 59 to approximately the center of the range of movement in the up and down, left and right, and front and back directions in order to facilitate the translational movement of the distal end part of the surgical instrument 24.

In addition to this, by the neutral assistance function FM7, force sense presentation may be performed to guide the attitude changing mechanism 51 to approximately the center of each range of movement for the pan direction turning manipulation Pa, the pitch direction turning manipulation Pi, and the roll direction turning manipulation Ro.

Further, in that case, the setting screen G1 may be configured to allow the surgeon to select a target manipulation for which the neutral assistance function is to be executed. For example, a configuration may be provided in which the pan direction turning manipulation Pa and the pitch direction turning manipulation Pi are not applied with the neutral assistance function, while the roll direction turning manipulation Ro is selectable for that function. This allows customization to suit the surgeon's preferences, as well as arrangements suitable for various situations.

In Fig. 16, as the wrist angle scaling function FM8, an example has been described in which the ratio selection field Sel4 is arranged to implement a scaling function for adjusting the amount of turning of the turning motion Pa' for the pan direction turning manipulation Pa, a scaling function for adjusting the amount of turning of the turning motion Pi' for the pitch direction turning manipulation Pi, and a scaling function for adjusting the amount of turning of the turning motion Ro' for the roll direction turning manipulation Ro.

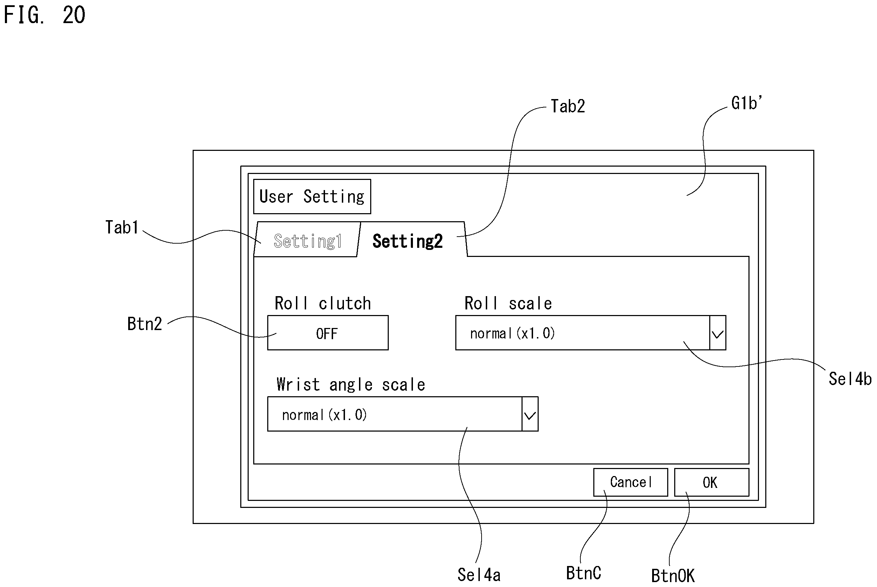

Fig. 20 illustrates an example of the wrist angle scaling function FM8 in which a selection field for scaling in the pan direction and pitch direction and a selection field for scaling in the roll direction are separate.

Specifically, on a setting screen G1b', a roll clutch button Btn2, a pan and pitch ratio selection field Sel4a, a roll ratio selection field Sel4b, a cancel button BtnC, and an execution button BtnOK are arranged.

It is possible to select different ratios in the pan and pitch ratio selection field Sel4a and the roll ratio selection field Sel4b, respectively. This allows customization to suit the surgeon's preferences, as well as arrangements suitable for various situations.

### <6. Summary>

A surgery assisting device in the surgery assisting system S described above is a master-side device 1 for remotely manipulating a slave-side device 2, and includes: a first manipulator (the grasping part 71 or the grasping mechanism 59) having a predetermined range of movement and used to manipulate a movable part (a movable part of the arm 22, a movable part of the surgical instrument holding device 23, and a movable part of the surgical instrument 24, that is, the slave-side drive part 100) of the slave-side device; a second manipulator (the clutch manipulator 76) provided corresponding to the first manipulator; and a control unit (the master-side control unit 93) configured to control the movable part in response to a manipulation input to the first manipulator, wherein when a manipulation is performed on the second manipulator, the control unit performs processing for moving a position of the first manipulator to a predetermined position (approximately the center of the range of movement) within the predetermined range of movement.

This processing is executed by the neutral assistance function FM7 described above.

This makes it possible to move, when the first manipulator is located near the end of the predetermined range of movement, the first manipulator to a position where it is easy for a surgeon to manipulate, specifically, to approximately the center of the range of movement.

Therefore, it is possible to prevent the surgeon from being able to perform the intended manipulation due to reaching the limit of the range of movement, making it easier to perform surgery. Further, it is possible to reduce manipulation errors based on the range of movement, which is suitable from the viewpoint of safety.

As described in Figs. 14 and 18, the master-side device 1 as a surgery assisting device may include a drive unit (the master-side drive unit 90) configured to feed back a tactile sensation detected in the slave-side device 2 via the first manipulator (the grasping part 71 or the grasping mechanism 59), and the control unit may control the drive unit to move the first manipulator to the predetermined position (e.g., approximately the center of the range of movement).

This makes it possible to gently guide the surgeon's hand and thus to move the first manipulator to a suitable manipulation position without damaging the surgeon's hand.

As described with reference to Fig. 12 and others, when the second manipulator (the clutch manipulator 76) is manipulated, the control unit (the master-side control unit 93) of the master-side device 1 as a surgery assisting device may remove the linkage between the first manipulator (the grasping part 71 or the grasping mechanism 59) and the first manipulator (the grasping part 71 or the grasping mechanism 59) used to manipulate the movable part (a movable part of the arm 22, a movable part of the surgical instrument holding device 23, a movable part of the surgical instrument 24, that is, the slave-side drive part 100).

This makes it possible to execute the processing of moving the first manipulator to the predetermined position after the linkage between the master-side device 1 and the slave-side device 2 is removed, which is suitable in terms of safety.

As described above, the first manipulator (the grasping part 71 or the grasping mechanism 59) of the master-side device 1 as a surgery assisting device may be a manipulator configured to move the movable part (a movable part of the arm 22, a movable part of the surgical instrument holding device 23, and a movable part of the surgical instrument 24, that is, the slave-side drive part 100) in axial directions of three axes (a turning axis in the pan direction, a turning axis in the pitch direction, and a turning axis in the roll direction) that are perpendicular to one another, and the predetermined position may be a center position of each range of movement in the axial directions of the three axes.

Specifically, the axial directions of the three axes are the front and back direction (the axial direction of the surgical instrument 24), the left and right direction (for forceps as the surgical instrument 24, the direction in which the distal end pieces 83 of the forceps are arranged side by side), and the up and down direction (the direction perpendicular to both the front and back direction and the left and right direction) when looking at the distal end part of the surgical instrument 24 in a direction in which the axis of the surgical instrument 24 extends. Then, the distal end part of the surgical instrument 24 is translated and moved to the center position of each range of movement so that the attitude of the distal end part does not change.

As described with reference to Fig. 17 and others, the control unit (the master-side control unit 93) of the master-side device 1 as a surgery assisting device may select whether or not to allow movement to the predetermined position in response to a predetermined operation input (e.g., an input operation to the neutral assistance button Btn1 on the setting screen G1a).

This allows the surgeon to switch on/off the neutral assistance function according to the surgeon's preferences. Therefore, even in a case where a plurality of surgeons use the master-side device 1, different settings can be made for each surgeon, making it possible to improve convenience.

As described in the modification example, the first manipulator (the grasping part 71 or the grasping mechanism 59) of the master-side device 1 as a surgery assisting device may be a manipulator configured to turn the movable part (a movable part of the arm 22, a movable part of the surgical instrument holding device 23, and a movable part of the surgical instrument 24, that is, the slave-side drive part 100) in directions around three axes that are perpendicular to one another, and the predetermined position may be a central angular position of each range of movement in the directions around the three axes.

As a result, when the neutral assistance function is used, not only translational manipulations but also turning manipulations are automatically adjusted to suitable positions, allowing surgery to be resumed at the optimal hand position and hand attitude.

As described with reference to Fig. 12 and others, the master-side device 1 as a surgery assisting device may include a continuous portion (the first coupling part 70) that is continuous with the grasping part 71 of the first manipulator, and the second manipulator (the clutch manipulator 76) may be provided in either the grasping part or the continuous portion.

This allows the surgeon to manipulate the second manipulator while performing translational movement and turning motion on the distal end part of the surgical instrument 24 while grasping the grasping part 71, making it possible to improve ease of manipulation.

As described with reference to Figs. 14 and 17 and others, the first manipulator (the grasping part 71 or the grasping mechanism 59) of the master-side device 1 as a surgery assisting device may include a right first manipulator (the grasping portion 71 or the grasping mechanism 59 of the right manipulation unit 4R) to be manipulated by the right hand of a user (surgeon or operator) and a left first manipulator (the grasping portion 71 or the grasping mechanism 59 of the left manipulation unit 4L) to be manipulated by the left hand of the user (surgeon), the second right manipulator may include a right second manipulator (the clutch manipulator 76 of the right manipulation unit 4R) corresponding to the right first manipulator and a left second manipulator (the clutch manipulator 76 of the left manipulation unit 4L) corresponding to the left first manipulator, and the control unit (the master-side control unit 93) may perform processing of moving positions of the left first manipulator and the right first manipulator to respective predetermined positions when the right second manipulator and the left second manipulator are manipulated together.

Providing a configuration to start the neutral assistance function in response to simultaneous manipulation of the clutch manipulators 76 on both hands makes it possible to prevent wrong manipulation.

As described with reference to Figs. 14 and 17 and others, the slave-side device 2 may include a right movable part (a movable part of the arm 22R, a movable part of the surgical instrument holding device 23 for the surgical instrument 24R, and a movable part of the surgical instrument 24R) corresponding to the right first manipulator (the grasping part 71 or the grasping mechanism 59 of the right manipulation unit 4R) and a left movable part (a movable part of the arm 22L, a movable part of the surgical instrument holding device 23 for the surgical instrument 24L, a movable part of the surgical instrument 24L) corresponding to the left first manipulator (the grasping part 71 or the grasping mechanism 59 of the left manipulation unit 4L), and the control unit (the master-side control part 93) may remove the linkage between the right first manipulator and the right movable part when the right second manipulator is manipulated, and remove the linkage between the left first manipulator and the left movable part when the left second manipulator is manipulated.

As a result, when the neutral assistance function is started in response to simultaneous manipulation of the clutch manipulators 76 on both hands, the linkage between the right first manipulator and the right movable part and the linkage between the left first manipulator and the left movable part are both removed (suspended) inevitably, which is suitable in terms of safety.

A surgery assisting device in the surgery assisting system S described above is a master-side device 1 for remotely manipulating a slave-side device 2, and may include: a first manipulator (the grasping part 71 or the grasping mechanism 59) having a predetermined range of movement and used to manipulate a movable part (a movable part of the arm 22, a movable part of the surgical instrument holding device 23, and a movable part of the surgical instrument 24, that is, the slave-side drive part 100) of the slave-side device 2; and a control unit (the master-side control unit 93) configured to control the movable part in response to a manipulation input to the first manipulator, wherein the control unit may perform processing of determining a correspondence relationship between an amount of manipulation of the first manipulator and an amount of movement of the movable part.

This makes it possible to move the corresponding movable part by a large amount by simply moving the first manipulator by a small amount.

Therefore, the surgeon can move the distal end part of the surgical instrument 24 as intended by moving the surgeon's wrist within its range of movement, making it possible to improve operability.

Furthermore, the number of times the above-described neutral assistance function is used can be reduced, making it possible to perform a smooth and quick surgery.

As described with reference to Figs. 14 and 18 and others, a correspondence relationship between an amount of manipulation of the first manipulator (the grasping part 71 or the grasping mechanism 59) and an amount of movement of the movable part (a movable part of the arm 22, a movable part of the surgical instrument holding device 23, a movable part of the surgical instrument 24, that is, the slave-side drive part 100) may be a relationship between an amount of manipulation of the first manipulator (the grasping part 71 or the grasping mechanism 59) and amounts of movement of turning of the movable part in the pan direction and the pitch direction.

This makes it possible to turn the distal end part of the surgical instrument 24 beyond the range of movement of the surgeon's wrist. Therefore, various manipulations can be performed easily.

As described with reference to Figs. 14 and 18 and others, a correspondence relationship between an amount of manipulation of the first manipulator (the grasping part 71 or the grasping mechanism 59) and an amount of movement of the movable part (a movable part of the arm 22, a movable part of the surgical instrument holding device 23, a movable part of the surgical instrument 24, that is, the slave-side drive part 100) may be a relationship between an amount of manipulation of the first manipulator and an amount of movement of turning of the movable part in the roll direction.

This allows the distal end part of the surgical instrument 24 to make a motion beyond the range of movement of the surgeon's wrist when the surgeon twists the wrist to turn the distal end part of the surgical instrument 24 in the roll direction.

As described with reference to Figs. 16 and 18 and others, the control unit (the master-side control unit 93) of the master-side device 1 as a surgery assisting device in the surgery assisting system S described above may perform display processing that allows a selection of correspondence relationships.

This allows the surgeon to select an amount of manipulation and amounts of movement in the pan direction and the pitch direction. Accordingly, the correspondence relationship can be customized according to the surgeon's preferences, which is suitable in a case where a plurality of surgeons use the master-side device 1.

As described with reference to Figs. 14, 16, and 18 and others, a display unit (the second monitor 8) may be included in which an image is displayed by display processing performed by the control unit (the master-side control unit 93) of the master-side device 1 as a surgery assisting device in the surgery assisting system S, and the control unit may display on the display unit a screen (the setting screen G1b) on which a selection field (the ratio selection field Sel4) that allows a selection of correspondence relationships is arranged, and determines a correspondence relationship in response to an operation on the selection field.

This allows the surgeon to perform a selection operation on the ratio selection field Sel4 arranged on the setting screen G1b displayed on the second monitor 8 and thus to change the correspondence relationship. Therefore, change operations are facilitated, and interference with surgery can be suppressed.

As described with reference to Fig. 16 and others, the control unit (the master-side control unit 93) of the master-side device 1 as a surgery assisting device in the surgery assisting system S may arrange a selection field including an option in which the amount of movement of the movable part (a movable part of the arm 22, a movable part of the surgical instrument holding device 23, a movable part of the surgical instrument 24, that is, the slave-side drive part 100) is larger than the amount of manipulation of the first manipulator (the grasping part 71 or the grasping mechanism 59).

This makes it possible to move the movable part beyond the range of movement of the surgeon's wrist. Therefore, a desired manipulation can be achieved with a single manipulation without removing the linkage between the master-side device 1 and the slave-side device 2, making it possible to improve convenience.

As described with reference to Fig. 16 and others, the control unit (the master-side control unit 93) of the master-side device 1 as a surgery assisting device in the surgery assisting system S may arrange a selection field including an option in which the amount of movement of the movable part (a movable part of the arm 22, a movable part of the surgical instrument holding device 23, a movable part of the surgical instrument 24, that is, the slave-side drive part 100) is smaller than the amount of manipulation of the first manipulator (the grasping part 71 or the grasping mechanism 59).

This makes it possible to move the movable part by a small amount even when the surgeon's wrist is moved significantly, making it possible to suitably respond to more precise manipulations. Therefore, it is possible to reduce manipulation errors, making it possible to improve surgical safety.

A surgery assisting device in the surgery assisting system S described above is a master-side device 1 for remotely manipulating a slave-side device 2, and may include: a first manipulator (the grasping part 71 or the grasping mechanism 59) having a predetermined range of movement and used to manipulate a movable part (a movable part of the arm 22, a movable part of the surgical instrument holding device 23, and a movable part of the surgical instrument 24, that is, the slave-side drive part 100) of the slave-side device 2; and a control unit (the master-side control unit 93) configured to control the movable part to turn in the roll direction in response to a specific manipulation on the first manipulator, wherein the control unit may perform processing of determining where or not to turn the movable part in the roll direction in response to the specific manipulation.

This makes it possible to remove the linkage between the master-side device 1 and the slave-side device 2.

In particular, a configuration is provided in which the linkage can be removed only for translational motion in a predetermined direction or turning motion in a direction around a predetermined axis, making it possible to provide manipulation modes that meet various needs of the surgeon and thus to improve convenience.

As described with reference to Figs. 14, 16, and 19 and others, in the master-side device 1 as a surgery assisting device in the surgery assisting system S, the specific manipulation on the first manipulator (the grasping part 71 or the grasping mechanism 59) may be a manipulation for turning the movable part (a movable part of the arm 22, a movable part of the surgical instrument holding device 23, a movable part of the surgical instrument 24, that is, the slave-side drive part 100) in the roll direction (the roll direction turning manipulation Ro), and the control unit (the master-side control unit 93) may determine whether or not to turn the movable part in the roll direction (the turning motion Ro') in response to the specific manipulation in the processing of determining (the processing of determining whether or not to move the movable part in response to a manipulation on the first manipulator).

As a result, when the surgeon twists the wrist to turn the distal end part of the surgical instrument 24 in the roll direction but the desired manipulation is disabled due to the range of movement of the wrist, it is possible to remove the linkage in the roll direction and to return the wrist to an attitude that makes it easy to manipulate accordingly. This makes it possible to provide an environment in which appropriate motions can be made when a large number of turning motions in the roll direction are desired.

As described with reference to Figs. 14, 16, and 19 and others, the control unit (the master-side control unit 93) of the master-side device 1 as a surgery assisting device in the surgery assisting system S may perform display processing that allows a selection of predetermined options, and when a predetermined option is selected, determine not to turn the movable part in response to the specific manipulation in the processing of determining (the processing of determining whether or not to move the movable part (a movable part of the arm 22, a movable part of the surgical instrument holding device 23, a movable part of the surgical instrument 24, that is, the slave-side drive part 100) in response to a manipulation on the first manipulator).

This allows the surgeon to select, for example, whether or not to perform a turning motion in the roll direction on the slave-side movable part in response to a turning manipulation in the roll direction. Accordingly, the correspondence relationship can be customized according to the surgeon's preferences, which is suitable in a case where a plurality of surgeons use the master-side device 1.

In particular, a configuration is provided that can be implemented by performing an operation on a display unit with a touch panel function, making it possible to achieve intuitive operations, which is preferable because wrong operations are less likely to occur.

As described with reference to Figs. 14, 16, and 19 and others, a display unit (the second monitor 8) may be included in which an image is displayed by display processing performed by the control unit (the master-side control unit 93) of the master-side device 1 as a surgery assisting device in the surgery assisting system S, and the control unit may display on the display unit a screen (the setting screen G1b) on which a selection operator (the roll clutch button Btn2) for selecting a predetermined option is arranged.

As a result, by performing a selection operation on the roll clutch button Btn2 arranged on the setting screen G1b displayed on the second monitor 8, the surgeon can change whether or not to make linkage between the master-side device 1 and the slave-side device 2 for a turning manipulation in the roll direction. Therefore, change operations are facilitated, and interference with surgery can be suppressed.

In particular, a configuration is provided that can be implemented by performing an operation on a display unit with a touch panel function, making it possible to achieve intuitive operations, which is preferable because wrong operations are less likely to occur.

As described with reference to Fig. 16 and others, as predetermined options, only a first option (Off option) for selecting to perform turning of the movable part (a movable part of the arm 22, a movable part of the surgical instrument holding device 23, a movable part of the surgical instrument 24, that is, the slave-side drive unit 100) in the roll direction and a second option (On option) for selecting not to perform the turning may be set, and the selection operator may be a button operator (the roll clutch button Btn2) that switches between a state in which the first option is selected and a state in which the second option is selected each time it is operated.

This makes it easy to change settings. Therefore, settings can be changed without interfering with the progress of the surgery.

Note that the various examples described above can be combined in any way.

### Reference Signs List

- 1: Master-side device
- 2: Slave-side device
- 59: Grasping mechanism (first manipulator, right first manipulator, left first manipulator)
- 70: First coupling part (continuous portion)
- 71: Grasping part (first manipulator, grasping part)
- 76: Clutch manipulator (second manipulator, right second manipulator, left second manipulator)
- 93: Master-side control unit (control unit)
- 100: Slave-side drive unit (movable part)

## Claims

1. A surgery assisting device that is a master-side device for remotely manipulating a slave-side device, the surgery assisting device comprising:
a first manipulator having a predetermined range of movement and used to manipulate a movable part of the slave-side device; and
a control unit configured to control the movable part to turn in a roll direction in response to a specific manipulation on the first manipulator,
wherein the control unit performs processing of determining where or not to turn the movable part in the roll direction in response to the specific manipulation.

2. The surgery assisting device according to claim 1, wherein
the control unit
performs display processing that allows selection of a predetermined option, and
when the predetermined option is selected, the control unit determines not to turn the movable part in response to the specific manipulation in the processing of determining.

3. The surgery assisting device according to claim 2, comprising a display unit in which an image is displayed by the display processing performed by the control unit,
wherein the control unit displays a screen on which a selection operator for selecting the predetermined option is arranged on the display unit.

4. The surgery assisting device according to claim 3, wherein
the predetermined options to be set include only a first option for selecting to perform turning of the movable part in the roll direction in response to the specific manipulation and a second option for selecting not to perform the turning, and
the selection operator is a button operator that switches between a state in which the first option is selected and a state in which the second option is selected each time the button operator is operated.
